# EUROPEAN PATENT SPECIFICATION

(11) **EP 0 492 560 B1**
(45) Date of publication and mention of the grant of the patent: **10.10.2001**
(21) Application number: 91122041.6
(22) Date of filing: 21.12.1991
(51) Int. Cl.: A61K 39/395, G01N 33/569, C12P 21/08, C12N 5/28

(54) **Human monoclonal antibodies directed against the transmembrane glycoprotein (gp41) of HIV-1, and related peptides**
Menschliche, monoklonale Antikörper gegen das transmembrane Glycoprotein (gp41) des HIV-1, und verwandte Peptide
Anticorps monoclonaux humains contre la glycoprotéine transmembranaire (gp41) de HIV-1, et peptides associés

(30) Priority: 26.12.1990 US 633964
(43) Date of publication of application: 01.07.1992
(60) Divisional application: 00102215.1
(73) Proprietor: BioClonetics Incorporated, Philadelphia, Pennsylvania 19147-3941 (US)
(72) Inventor: Cotropia, Joseph P., Philadelphia, Pennsylvania 19147 (US)
(74) Representative: UEXKÜLL & STOLBERG

(56) References cited:
- EP-A- 0 316 495
- WO-A-90/15071
- JOURNAL OF VIROLOGY vol. 61, no. 8, August 1987, WASHINGTON, USA pages 2639 - 2641 GNANN ET AL 'Fine mapping of an immunodominant domain in the transmembrane glycoprotein of human immunodeficiency virus'
- JOURNAL OF VIROLOGY vol. 64, no. 9, September 1990, WASHINGTON, USA pages 4123 - 4129 BUGGE ET AL 'Analysis of a highly immunodominant epitope in the Human Immunodeficiency Virus type 1 transmembrane glycoprotein,gp41,defined by a human monoclonal antibody'
- CHEMICAL ABSTRACTS, vol. 109, 1988, Columbus, Ohio, US; abstract no. 91111, ROSEN ET AL 'Synthetic HTLV-III peptides,compositions and kits containing them,and their use in immunoassays,immunization,and antibody production' page 547 ;column 2 ;
- CHEMICAL ABSTRACTS, vol. 111, 1989, Columbus, Ohio, US; abstract no. 230559, NORRBY ET AL 'STLV-III (simian T-lymphotrophic virus type III)-related polypeptides,diagnostic systems,and assay methods' page 606 ;column 1 ;
- J Inf Dis 156 (1987) 261-7

## Description

### FIELD OF THE INVENTION

This invention relates to anti-HIV-1 monoclonal antibodies and specifically to monoclonal antibodies which bind to a viral epitope, thereby neutralizing the virus. The invention also relates to continuous cell lines capable of producing the antibodies. The antibodies of this invention are useful for diagnosis, prognosis, prophylaxis and therapy. This invention also relates to prognostic tests for viral diseases, and particularly prognostic tests for Acquired Immunodeficiency Syndrome (AIDS).

### BACKGROUND OF THE INVENTION

The human immunodeficiency virus (HIV-1) has been established as the primary etiologic agent in the pathogenesis of acquired immunodeficiency syndrome (AIDS) and related disorders. (Barre-Sinoussi, et al. Science (1983) 220:868-871; Gallo, et al., Science (1984) 224:500-503; Levy, et al., Science (1984) 225:840-842).

The CD4+ cells play a central role in HIV infection. (Fauci, Science (1988) 239:617-622). CD4 is a molecule present on the surface of certain lymphocytes and, to a lesser degree, macrophages. The CD4 molecule plays a significant role in the function of T4 helper lymphocytes and serves as a marker for such cells. (Gallo, R.C. and Montagnier, L., Scientific American (1988) 259:41-48.) The virus uses the CD4 receptor to gain entry into a number of cells. (Dalgleish, et al., Nature (1984) 312:763-767). The envelope glycoprotein, gp160, is the precursor to the gp120, which specifically binds to the surface receptor (CD4) of CD4+ cells, and the gp41, the transmembrane (TM) glycoprotein which initiates cell-membrane fusion, leading to the formation of multinucleated giant cells commonly called syncytia. (Kowalski, Science (1987) 237:1351-1355). Fusion leads to the death of the syncytial cells. While HIV-1 may also cause cell death through mechanisms independent of cell fusion, data suggest that the formation of syncytia contributes to the progressive depletion of CD4+ cells (T4 helper lymphocytes), quantitatively and functionally. (Lifson, et al., Nature (1986) 323:725-728). This is the most profound hematologic feature and hallmark associated with acquired immunodeficiency syndrome (AIDS) (Broder, S.M. and Gallo, R.C., N. Eng. J. Med. (1984) 311:1292-1297), as demonstrated by impaired cell-mediated immunity.

Infection of humans with HIV-1 leads to a humoral immune response by B lymphocytes resulting in the production of antibodies directed against most of the viral structural antigens. A particular subset of antibodies is directed against HIV envelope antigens (gp120 and gp41) which may be involved in induction of active immunity. (Matthews, et al., AIDS Research and Human Retroviruses (1987) 3:197-206). Neutralization assays with sera from HIV-infected individuals (Robert-Guroff, et al., Nature (1985) 316:72-74; Rasheed, et al., Virology (1986) 150:1-9) or from immunized animals, suggest that the envelope glycoprotein contains epitope(s) that elicit antibodies capable of neutralizing HIV infection in vitro. As an in vivo corollary, it has been demonstrated that high neutralizing antibody titers correlated with a better clinical outcome, and low or decreasing neutralizing antibody titer signaled poor prognosis. (Robert-Guroff, et al., AIDS Research and Human Retroviruses (1988) 4:343-350). A decrease in average antibody titers has been clinically observed in late stages of infection, particularly with regard to antibodies directed against the HIV envelope epitopes and specifically against the TM gp41 region containing the amino acid sequence against which the herein described human monoclonal antibody is biologically reactive. (Shafferman, et al., AIDS Research and Human Retroviruses (1989) 5:33-39; Chiodi, et al., J. Med. Virol. (1987) 23:1-9; McPhee, et al., FEBS Lett. (1988) 233:393-396).

Measures capable of boosting the neutralizing antibody titer of individuals already infected with the virus, eliciting high-titer neutralizing antibodies (i.e., active immunotherapy), or increasing neutralizing antibodies (i.e., passive immunotherapy) in individuals at risk would prove beneficial in controlling viral spread in vivo or in preventing new infection. (Robert-Guroff, et al., AIDS Research and Human Retroviruses (1988) 3:343-350).

The present invention makes possible the measures cited above. Any attempts at passive immunotherapy will require the production of large quantities of antibody on a routine basis. The development of a continuous cell line accommodates this. The monoclonality of the antibody enables the administration of reactive physiological amounts of the antibody since all of the antibody being administered is directed against the biologically active epitope of the virus, unlike polyclonal serums which contain antibodies against other structural proteins as well. Those skilled in the art will recognize that a mouse monoclonal antibody directed against HIV-1 epitopes would have the same effect as the human monoclonal antibody, however, with possible therapeutical side effect limitations as a result of being xenogeneic. The potential immunogenicity of the peptide sequence of this epitope, as evidenced by the immunogenicity of a peptide of similar sequence, will enable safe and effective vaccination of individuals, thereby avoiding the great risks involved in immunizing with attenuated or even killed viruses.

The present invention is further directed to a kit and a method for detecting the presence and determining concentration of an antibody that inhibits HIV-1 **fusion-associated epitope,** a peptide on gp41 with the amino acid sequence represented by **GCSGKLIC** (Seq. No. 1). The detection and quantitation method includes an enzyme-linked immunosorbent assay (ELISA).

The determination of the concentration of the antibody that inhibits HIV-1 fusion-associated epitope on gp41, present in body fluids of a patient seropositive for HIV-1 antibodies, provides the physician with an objective means to form a prognosis for each individual case and assists the clinician therefore in determining the appropriateness to initiate medical intervention or change therapy.

### SUMMARY OF THE INVENTION

The present invention is concerned with a novel human monoclonal antibody which defines and neutralizes a biologically functional antigenic/immunogenic site on the HIV-1 transmembrane (TM) envelope glycoprotein.

The invention of the human monoclonal antibody, the production of the antibody, the identification of the epitope (peptide) to which the antibody binds, and the delineation of the biologically important function of the defined epitope, are achieved and described in detail by the following outlined immunochemical methods and biological assays.

The antigenic/immunogenic peptide identified as the epitope (target antigen) is contained within the twelve amino acid (L-form) sequence: leucine-glycine-isoleucine-tryptophan-glycine-cysteine-serine-glycine-lysine-leucine-isoleucine-cysteine (Seq. No. 2). Since antigenic determinants have been reported to be represented by as few as five amino acid residues, the actual epitope may be a truncation of this sequence. (Barrett, J.T., Textbook of Immunology (1983) p. 41). Specifically, the human monoclonal antibody immunochemically binds to a conserved peptide of the HIV-1 transmembrane (TM) glycoprotein designated gp41, and, as a consequence of this antibody-antigen reaction, biologically blocks syncytia formation between HIV-1 virally infected human lymphocytes and uninfected lymphocytes (CD4+ cells).

The described human monoclonal antibody binds to the identified viral epitope, specifically leucine-glycine-isoleucine-tryptophan-glycine-cysteine-serine-glycine-lysine-leucine-isoleucine-cysteine (Seq. No. 2), and the fusion of HIV-1-infected CD4+ cells with non-infected CD4+ cells is thereby blocked. Cell fusion is a biologically functional functional process that can therefore be ascribed to this conserved immunodominant region.

The human monoclonal antibody described (Anti-gp41) inhibits fusion of CD4+ cells caused by HIV-1 and therefore blocks infection via binding to the gp41 transmembrane glycoprotein, specifically the fusion-associated epitope as delineated. This inhibition signifies the utility of Anti-gp41 in passive immunotherapy. Administration of the retrovirus/vaccine containing the immunogenic fusion-associated epitope/subunit will elicit fusion blocking antibody (as evidenced by the human B cell line identified from the HIV-1-infected, naturally immunized, patient/donor), thereby providing protection against the infection and depletion of CD4+ cells and, therefore, preventing the development of acquired immunodeficiency syndrome.

### BRIEF DESCRIPTION OF THE DRAWINGS

For a more complete understanding of the present invention and for further advantages thereof, reference is made to the following Detailed Description taken in conjunction with the accompanying Drawings, in which:
FIGURE 1 is a depiction of the Western blot described in Example 3;
FIGURE 2 is a graph of the results of the syncytium-forming microassay described in Example 11;
FIGURE 3 presents the 23 amino acid peptide sequences for SIV, used in the ELISA by Johnson et al., as reported in AIDS Research and Human Retroviruses (1988) 4:159-164, and the analogous regions for HIV-2 and HIV-1;
FIGURE 4 illustrates a linear comparison of the peptide sequences tested in determining the specificity of Anti-gp41;
FIGURE 5 presents the linear representation of amino acid sequences (three letter abbreviation) contained within the transmembrane glycoprotein gp41 of HIV-1 for Peptide 2 and Peptide 6120;
FIGURE 6 illustrates the fine epitope mapping for Peptides 232-240;
FIGURE 7 presents the linear representation of amino acid sequences (three letter abbreviation) contained within the transmembrane glycoprotein gp41 of HIV-1 for Peptide 237 and Peptide 238;
FIGURE 8 shows the reduced and non-reduced Western Blot for the donor patient plasma and for Clone 3 antibody; and
FIGURE 9 illustrates immunofluorescence profiles by flow cytometry for HIV-infected Sup-T1 cells and uninfected Sup-T1 cells.

### DETAILED DESCRIPTION OF THE INVENTION

The subject invention is to novel human monoclonal antibodies that are biologically reactive against a specific immunodominant peptide region within the transmembrane glycoprotein gp41 envelope component of the human immunodeficiency virus (HIV-1), delineation of the peptide region (epitope) to which the human monoclonal antibody binds, and immunologic description of the associated biological function of the epitope.

The present invention also relates to a human monoclonal antibody, which requires the amino acid sequence Leu-Ile-Cys as the minimal essential epitope. Further encompassed is a Fab, F(ab')₂, or Fv fragment of the monoclonal antibody of the invention.

The invention also provides a human hybridoma cell line producing the monoclonal antibody. Preferably, the human hybridoma cell line has the ATCC deposit No. ATCC-CRL-10198.

The following describes the creation of cells which are capable of producing, in vitro, ad infinitum, human monoclonal antibodies that are biologically active against the transmembrane glycoprotein gp41 of HIV-1.

Based on the fact that mononuclear cells circulating in the peripheral blood of HIV-1-infected individuals produce antibodies against HIV antigens, an asymptomatic patient who had high titers of plasma antibodies against HIV was selected as a donor of B-lymphocytes. The B-lymphocytes collected from this patient were subsequently transformed by Epstein-Barr Virus (EBV). Seven anti-HIV antibody-producing human lymphoblastoid cell lines have been obtained in this manner and have been stable with respect to antibody production. In order to further assure stability and augment antibody production, an aliquot of each of the created EBV transformed human cell lines has been fused with a heteromyeloma (mouse-human hybrid myeloma) as a fusion partner to produce a hybridoma counterpart.

Supernatants from the resultant hybridomas were screened for antibodies against HIV. Cell lines testing positive for anti-HIV antibody production were subcloned twice and secreted antibody was then tested specifically for binding against the envelope glycoproteins of the virus (i.e., gp41 and gp120). One of seven human monoclonal antibodies testing positive for anti-gp41 reactivity (designated Clone 3 antibody) has been purified from the cell culture supernatants by affinity chromatographic techniques. This human monoclonal antibody (not an animal derived monoclonal antibody) has been demonstrated to have biological activity at physiologic concentrations against HIV-1 (specifically HTLV-IIIB) in a fusion (syncytium) inhibition, in vitro assay; and in neutralization of free HIV-1 (specifically SF2) infectivity of human mononuclear cells in culture. The specific level of fusion inhibition and inhibition of free virus infectivity achieved is detailed in Example 12 and Example 19, respectively, discussed hereinafter.

Development of Clone 3 cell line is detailed further in Example 1 discussed hereinafter. Clone 3 was deposited with the American Type Culture Collection (ATCC) in Rockville, Maryland on August 10, 1989 and received accession number ATCC CRL 10198.

Additionally, the specific amino acid sequence (epitope) to which the human monoclonal antibody binds has been ascertained, thereby ascribing the associated biological function of cell fusion to the epitope (fusion-associated epitope). Epitope mapping is discussed hereinafter in Example 8, Example 9, and Example 10.

The biological reactivity of the monoclonal antibody and the biological function of the epitope to which it binds reveal the invention's utility for passive therapeutic intervention in the treatment of acquired immunodeficiency syndrome. Specifically, data to support the efficacy of passive immunotherapy in chimpanzees have been published, for it has been determined that neutralization of in vivo HIV-1 infectivity can be mediated by in vitro neutralizing antibody directed against the gp120 major, yet hypervariable, neutralizing epitope. (Emini, et al., V. International Conference on AIDS (1989), Abstract No. Th.C.O.30, p. 538). The human monoclonal antibody can be administered to patients who lack neutralizing antibodies against this epitope within gp41, thereby providing passive immunotherapy. In a parallel human study, data from recent clinical trials (Jackson, et al., Lancet (1988) 2:647-652; Karpas, A., Proc. Natl. Acad. of Sciences (U.S.A.) (1988) 85:9234-9237) have demonstrated that passive immunization improved the status of patients with advanced AIDS. In those trials, passive immunization was accomplished by transfusing plasmas containing antibodies from asymptomatic AIDS patients into the symptomatic AIDS recipients.

In a similar approach, another therapeutic use of the monoclonal antibody of the present invention is the active immunization of a patient using an anti-idiotypic antibody raised against the monoclonal antibody of the present invention. Immunization with an anti-idiotype which mimics the structure of the fusion-associated epitope could elicit an active anti-gp41 response. (Linthicum, D.S. and farid, N.R., Anti-Idiotypes, Receptors , and Molecular Mimicry (1988), pp. 1-5 and 285-300).

According to another embodiment, the invention relates to an immunoassay method for the detection and quantitation of HIV infection comprising:
(a) incubating a sample suspected to be infected with HIV with a human monoclonal antibody or a fragment thereof; and
(b) detecting the binding of the antibody to the sample via immunological methods.

Further encompassed is an immunoassay method for the detection and quantitation of antibodies directed against gp41 of HIV-1 and prognosis of HIV-1 conditions comprising:
(a) coating a solid support with an effective amount of a peptide composition comprising at least a portion of a peptide selected from the group consisting of:

   (I) X-R-Leu-Ile-Cys-R'-Y-Z

   where X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or Cys; Z is OH or NH₂; and R is either absent or a sequence of 1-5 amino acids; and R' is either absent or a sequence of 1-2 amino acid; analogues thereof wherein the amino acids in the sequence are substituted as long as the immunoreactivity with antibodies to HIV-1 gp41 derived from the three-dimensional conformation of the sequence is substantially preserved; and mixtures and polymers thereof as the antigen, linear as well as cyclic;
(b) adding a test sera, and in parallel testing adding a known and quantitated amount of a human monoclonal antibody or a fragment thereof diluted with a buffer wherein the antibodies to the peptide in the test sera and the quantitated human monoclonal antibody standard control form a peptide-antibody complex with the peptide composition;
(c) incubating the mixture at an appropriate temperature and time;
(d) detecting the presence of the peptide-antibody complex and determining the concentration of the specific antibody via the generated standard curve; and
(e) making a good prognosis that said patient is unlikely to develop the symptoms of AIDS based upon a finding of an amount of an antibody binding to the Peptide (I), and a poor prognosis that said patient is likely to develop the symptoms of AIDS based upon a finding of no antibody finding to the Peptide (I).

In this immunoassay method, the solid support is coated with the peptide preferably in an amount of 0.1-20 micrograms.

According to another embodiment, step (d) of the immunoassay method comprises introducing a second known antibody labeled with an enzyme and a substrate which reacts with the enzyme to form a colored product. Preferably, the second known antibody labeled with a radioactive element.

Further provided is a test kit for the detection and quantitation of antibodies directed against gp 41 of HIV-1 and prognosis of HIV-1 condition comprising:
(a) a solid support;
(b) coated onto the solid support, an immunoabsorbent comprising a peptide composition according to claim 6(a):
(c) a sample of normal serum as negative control;
(d) a sample of quantitated affinity-chromatography purified human monoclonal antibody or a fragment thereof as positive control and for construction of a standard curve;
(e) a buffer for diluting the test fluid (e.g. serum samples) the antibody; and
(f) a second antibody tracer to bind with the antibody-antigen complex formed in the immunoassay.

The present invention relates to a method for neutralizing the retrovirus HIV-1 in vitro, comprising adding to a mixture of HIV-1 virus and uninfected cells, an effective amount of the monoclonal antibody or a fragment thereof under conditions effective for allowing said neutralizing agent to neutralize HIV-1.

Further provided is a method for producing a pharmaceutical composition for effecting passive immunization comprising the step of mixing the monoclonal antibody or a fragment thereof with a pharmaceutically acceptable carrier. In adition, the invention relates to a method for producing a pharmaceutical composition for effecting antiviral therapy through selective cellular toxicity comprising conjugating of a cytotoxic agent to the monoclonal antibody or a fragment thereof.

According to another embodiment, a method for producing an anti-idiotipic antibody is provided which comprises the step of injecting an immunogenic amount of the monoclonal antibody or a fragment thereof into an animal.

The peptide sequence set forth below in a general formula is common to HIV-1, HIV-2, and SIV:
X-a-b-c-tryptophan-glycine-cysteine-x-x-x-x-x-cysteine-Y-Z.

The specific peptide sequence, and truncated sequences, for each of the comparable analogous conserved immunodominant regions from HIV-1, HIV-2, and SIV are set forth in the following formulae.

For the Human Immunodeficiency Virus-1 (HIV-1), a conserved immunodominant, antigenic/immunogenic twelve amino acid residue peptide (amino acids numbers 598-609, Gnann numbering system, id.), a structural component of the transmembrane glycoprotein gp41, is immunologically reactive with the HIV-1 specific fusion blocking human monoclonal antibody. The novel peptide sequence and the truncated sequences are set forth in the formula below:
X-a₁-b₁-c₁-tryptophan-glycine-cysteine-¹x₁-²x₁-³x₁-⁴x₁-⁵x₁-cysteine-Y-Z where
X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or cysteine; and Z is OH or NH₂, and
a₁ is leucine
b₁ is glycine
c₁ is isoleucine
¹x₁ is serine
²x₁ is glycine
³x₁ is lysine
⁴x₁ is leucine
⁵x₁ is isoleucine

Alternatively, a truncated peptide can be produced where a is present, with b and c also being present, to represent the original 12-mer; a is absent, with b and c being present, to represent an 11-mer; a and b are absent, with c only being present, to represent a 10-mer; a and b and c are absent, to represent a 9-mer, depicted by the following sequence formula: tryptophan-glycine-cysteine-x-x-x-x-x-cysteine which is common to analogous positions within the three comparable epitopes for HIV-1, HIV-2 and SIV. Similarly, the tryptophan and glycine residues may also be deleted resulting in the production of an 8-mer and 7-mer, respectively.

For the Human Immunodeficiency Virus-2 (HIV-2), a conserved immunodominant, antigenic/immunogenic twelve amino acid residue peptide (amino acids numbers 592-603, Franchini numbering system as reported by Johnson, et al., AIDS Research and Human Retroviruses (1988) 4:159-164) which is a structural component of the transmembrane glycoprotein, and the truncated sequences are set forth in the formula below:
X-a₂-b₂-c₂-tryptophan-glycine-cysteine-¹x₂-²x₂-³x₂-⁴x₂-⁵x₂-cysteine-Y-Z where
X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or cysteine; and Z is OH or NH₂, and
a₂ is leucine
b₂ is asparagine
c₂ is serine
¹x₂ is alanine
²x₂ is phenylalanine
³x₂ is arginine
⁴x₂ is glutamine
⁵x₂ is valine

Alternatively, a truncated peptide can be produced where a is present, with b and c also being present, to represent the original 12-mer; a is absent, with b and c being present, to represent an 11-mer; a and b are absent, with c only being present, to represent a 10-mer; a and b and c are absent, to represent a 9-mer as described above. The tryptophan and glycine residues may also be deleted resulting in the production of an 8-mer and 7-mer, respectively.

For the simian immunodeficiency virus (SIV), a conserved immunodominant, antigenic/immunogenic twelve amino acid residue peptide (amino acids numbers 617-628, Franchini numbering system, id.), which is a structural component of the transmembrane glycoprotein gp32, and the truncated sequences are set forth in the formula below:
X-aₛ-bₛ-cₛ-tryptophan-glycine-cysteine-¹xₛ-²xₛ-³xₛ-⁴xₛ-⁵xₛ-cysteine-Y-Z where
X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or cysteine; and Z is OH or NH₂, and
aₛ is leucine
bₛ is asparagine
cₛ is alanine
¹xₛ is alanine
²xₛ is phenylalanine
³xₛ is arginine
⁴xₛ is glutamine
⁵xₛ is valine

Alternatively, a truncated peptide can be produced where a is present, with b and c also being present, to represent the original 12-mer; a is absent, with b and c being present, to represent an 11-mer; a and b are absent, with c only being present, to represent a 10-mer; a and b and c are absent, to represent a 9-mer as described above. The tryptophan and glycine residues may also be deleted resulting in the production of an 8-mer and 7-mer, respectively.

The three-letter and single-letter abbreviations for the amino acids are as follows:
Ala(A), alanine
Arg(R), arginine
Asn(N), asparagine
Asp(D), aspartic acid
Cys(C), cysteine
Gln(Q), glutamine
Glu(E), glutamic acid
Gly(G), glycine
His(H), histidine
Ile(I), isoleucine
Leu(L), leucine
Lys(K), lysine
Met(M), methionine
Phe(F), phenylalanine
Pro(P), proline
Ser(S), serine
Thr(T), threonine
Trp(W), tryptophan
Tyr(Y), tyrosine
Val(V), valine

### Description of Non-Human Primate and Human Clinical Subunit Vaccine Trials

The only animal that can be reproducibly infected with HIV, thus providing an experimental system for testing the effectiveness of prototype vaccines, is the chimpanzee. Although chimpanzees can be experimentally infected with HIV-1, clinical disease has not, to date, developed in infected animals. Furthermore, the supply of chimpanzees available for biomedical research is limited since the assignation of the chimp as an endangered species. The recently described simian immunodeficiency virus (SIV, STLV-III), Desrosiers, R.C. and Letvin, N.L., Rev. Infect. Dis. (1987) 9:438-446, provides a potentially more useful model system based on the infection of rhesus macaques and African green monkeys (Kanki, et al., Science (1985) 230:951-1954).

SIV, although closely related to HIV-1 (Hirsch, et al., Cell (1987) 49:307-319; Franchini, et al., Nature (1987) 328:539-543; Chakrabarti, et al., Nature (1987) 328:543-547), is genetically more related to HIV-2 (Franchini, et al., supra, Chakrabarti, et al., supra), which also causes human AIDS (Clavel, et al., N. Eng. J. Med. (1987) 316:1180-1185). And, most importantly, SIV induces clinical AIDS similar to the human syndrome in infected macaques. (Desrosiers, R.C. and Letvin, N.L., supra).

The serologic diagnosis of SIV infection in monkeys has been made using traditional antibody assays, including enzyme-linked immunosorbent assay (ELISA) with whole-virus lysate as antigen. For the serologic diagnosis of HIV-1 infection in humans, a more sensitive, specific, as well as simple, diagnostic method has been investigated in ELISA systems using synthetic peptides as solid-phase antigens (site-directed ELISA). In particular, selected synthetic peptides that correspond to sequences from the amino-terminal half (amino acids 586-620) of the transmembrane glycoprotein (gp41) have reacted with over 99% of sera from human AIDS patients (Wang, et al., Proc. Natl. Acad. Sci. (U.S.A.) (1986) 83:6159-6163; Smith, et al., J. Clin. Microbiol. (1987) 25:1498-1504; Gnann, et al., Science (1987) 237:1346-1349; Gnann, et al., J. Infect. Dis. (1987) 156:261-267; Chiodi, et al., J. Med. Virol. (1987) 23:1-9).

A synthetic peptide from an analogous region of the SIV transmembrane glycoprotein (gp32) is highly immunoreactive with sera from SIV-infected primates. This reactivity extends across four primate species from three genera and indicates infection by at least two distinct isolates of SIV in experimentally and naturally infected monkeys. (Johnson, et al., AIDS Research and Human Retroviruses (1988) 4:159-164.) Preliminary experiments also demonstrated that this peptide from the SIV gp32 reacted strongly with sera from humans infected with HIV-related West African viruses HIV-2 (HTLV-IV). Furthermore, reactivity with this peptide was specific for infection with the West African viruses, since these same sera did not react with the analogous peptides from HIV-1. (Norrby, et al., Nature (1987) 329:248-250.)

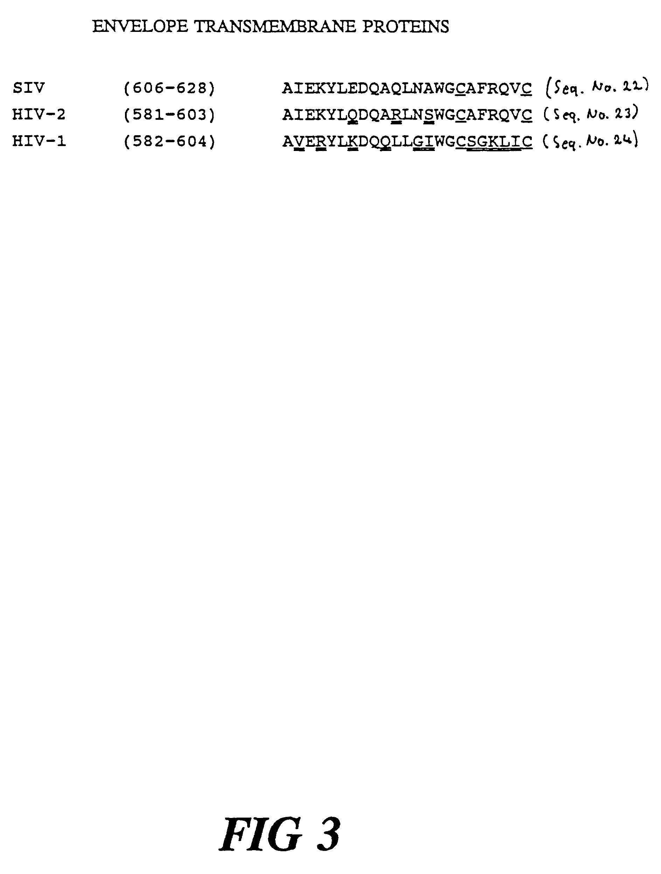

Sequences of the synthetic peptide used in the ELISA to detect antibodies against the SIV transmembrane glycoprotein (gp32) and the analogous regions from HIV-2 and HIV-1 are presented in FIGURE 3, using the single-letter abbreviations for the amino acids. The sequences are numbered according to the Johnson reference, supra. It should be noted that the sequence for HIV-1 contains the 12 amino acid sequence of peptide 2 (amino acid residues 593-604 in the Franchini numbering system) described hereinafter in Example 8 and used in epitope mapping to determine the specificity for the human monoclonal antibody Anti-gp41 produced by Clone 3.

Non-identical amino acid residues for HIV-1 and HIV-2 compared with the analogous regions with the SIV sequence are denoted by double underlining. The two cysteine amino acid residues are marked by a singly underlined C.

The three analogous peptide sequences delineated above, consisting of 23 amino acid residues, have some unique biochemical structural features that are common to each. Two closely spaced cysteine residues may serve to orient the peptide in a similar configuration in order that an essential biological function can proceed, perhaps via disulfide bonding. The importance of these cysteine residues for maintaining antigenicity has been demonstrated recently for a similar HIV-1 peptide.

Sequential single amino acid deletions from the amino terminus of the 12 amino-acid peptide (amino acid residues 598-609 in the Gnann numbering system, which corresponds to amino acid residues 593-604 in the Franchini numbering system) of gp41 revealed a minor reduction in recognition by HIV-1 positive (polyclonal) sera in ELISA from 100% reactivity to 95%, 91%, and 86% activity when the amino acids leucine, glycine, and leucine, respectively, were specifically removed in a stepwise fashion.

Removal of the next two amino acid residues, tryptophan and glycine, which occupy analogous positions within both comparable epitopes for HIV-2 and SIV fitting the sequence formula tryptophan-glycine-cysteine-x-x-x-x-x-cysteine, produced eight and seven-residue oligopeptides (8-mer and 7-mer) that were reactive with only 64% and 48% of (polyclonal) sera from HIV-positive individuals, respectively. Thus, the essential epitope for immune recognition is determined as the 7-amino-acid sequence containing 2 cysteine residues (amino acids 603-609).

The smallest peptide retaining a degree of reactivity (with less than a 30% decrease in reactivity when compared to the original 12 amino acid sequence (12-mer)) was that structure with the common amino acid sequence tryptophan-glycine-cysteine-x-x-x-x-x-cysteine, or a nine amino acid peptide (9-mer). (Gnann, et al., J. Virol. (1987) 61:2639-2641).

Importantly, this region from HIV-1 is immunogenic as well as antigenic. Immunization of New Zealand white rabbits with the synthetic peptide leucine-glycine-leucine-tryptophan-glycine-cysteine-serine-glycine-lysine-leucine-isoleucine-cysteine (Seq. No. 28) (amino acid residues 598-609) induced high-titers of binding polyclonal antibodies. (Gnann, et al., J. Infect. Dis. (1987) 156:261-267.) No data characterizing biological function of the elicited antibodies were disclosed.

The unique and important characteristic of the described invention is that: a human B-lymphocyte cell line from an individual exposed to HIV-1 (naturally immunized by infection) has been established and immortalized, and is producing a biologically active fusion-blocking human monoclonal antibody directed against an epitope, within the peptide region described, that being specifically within the amino acid sequence leucine-glycine-isoleucine-tryptophan-glycine-cysteine-serine-glycine-lysine-leucine-isoleucine-cysteine (Seq. No. 2). This is believed to be the first report of human monoclonal antibodies that have been demonstrated to have fusion-blocking biological activity, as determined by syncytia inhibition assays, against HIV-1 gp41. Although other investigators have developed human monoclonal antibodies against gp41, these antibodies have no fusion-blocking blocking biologic activity. (Banapour, et al., J. Immunology (1987) 139:4027-4033; Gorny, et al., Proc. Natl. Acad. Sci. (U.S.A.) (1989) 86:1624-1628; Kawamura, et al., V. International Conference on AIDS (1989), Abstract No. Th.C.O.4, p. 533; Boyer, et al., V. International Conference on AIDS (1989), Abstract No. T.C.P.59, p. 576; Zolla-Pazner, et al., V. Int'l Conf. on AIDS (1989), Abstract No. Th.C.O.10, p. 534).

Furthermore, computer modeling of the HIV-1 envelope protein sequence has predicted that the two amino acids (Ser-Gly) between the two cysteine residues in question participate in a B turn in the loop, hypothesized to be external to the viral membrane and formed by disulfide bonding between the two cysteines. (Johnson, et al, supra, p. 164, reporting on computer-modeling performed by Modrow, et al., J. Virol. (1987) 61:570-578). Thus, this region, which is also conserved among numerous isolates of HIV-1 (WMJ1, WMJ2, WMJ3, BH10, ARV2, LAV1A, HAT3), has distinctive biochemical and immunogenic structural properties that allow for a stable antigenic configuration, and, most importantly, also provides the essential biological fusion-associated function necessary for the virus to maintain infectiousness.

### Example 1

### Production of Cell Lines Synthesizing Human Monoclonal Antibodies to HIV

Peripheral blood, collected from a seropositive individual who had high titers of antibodies to HIV-1, was heparinized, centrifuged to remove plasma, then diluted 1:1 with phosphate buffered saline (PBS), pH 7.4, and subjected to Ficoll-Hypaque gradient centrifugation to obtain mononuclear cells. Approximately 1 x 10⁷ mononuclear cells at the gradient interface were recovered, washed thrice with PBS and then incubated for 1.5 hours at 37°C in 3 ml of filtered (0.45 micron) culture supernatant from EBV transformed marmoset cell line B95-8 containing 100,000 transforming units per ml. Cyclosporin A was then added at a final concentration of 2 micrograms/ml to inhibit suppressor T cells.

Lymphocytes were cultured in 1.5 ml of Iscove Modified Dulbecco's Medium (Iscove, N.N. and Melchers, F., J. Exp. Med. (1978) 147:923-928) supplemented with 10% (vol/vol) fetal bovine serum (HyClone), L-glutamine (2 mM), penicillin (100 units/ml), streptomycin (100 micrograms/ml), and fungizone (0.25 micrograms/ml) media of 1 x 10⁶ cells/ml, in an atmosphere of 5% carbon dioxide at 37°C.

After screening for anti-HIV antibody production by a commercial Enzyme-linked Immunosorbent Assay (ELISA) (Genetic Systems, Seattle, WA), positive culture wells were cloned by limiting dilution in 96-well, U-bottom plates at 1 cell per well on a feeder layer of irradiated (3000R) human lymphocytes (20,000 cells/well).

An aliquot of lymphoblastoid cells testing positive for anti-HIV production was expanded in 25 cm² flasks, 7 ml/flask, (Costar) and cultured for an additional week to a concentration of approximately 1 x 10⁶ cells/ml. Lymphoblastoid cells were then fused by polyethylene glycol (PEG 50%, MW 1000) with heteromyelomas (HAT sensitive, ouabain resistant) in a ratio of 2:1 for preparation of hybridomas. (Bartal, A.H. and Hirshaut, Y. Methods of Hybridoma Formation (1987)).

Cell lines with supernatants testing positive for anti-HIV antibodies were then subcloned twice by limiting dilution at 0.3 cells per well in 96-well, U-bottom microtiter plates, to assure monoclonality of the produced antibody. Each cell line has been cryopreserved in liquid nitrogen to form a master cell bank (MCB). The cells were frozen at a concentration of 5 x 10⁶ cells/ml in a freezing solution of Iscove media supplemented with 20% fetal bovine serum and 10% dimethylsulfoxide, 1 ml/ampule.

### Example 2

### Human Monoclonal Antibody Screening By Enzyme-Linked Immunosorbent Assays (ELISA)

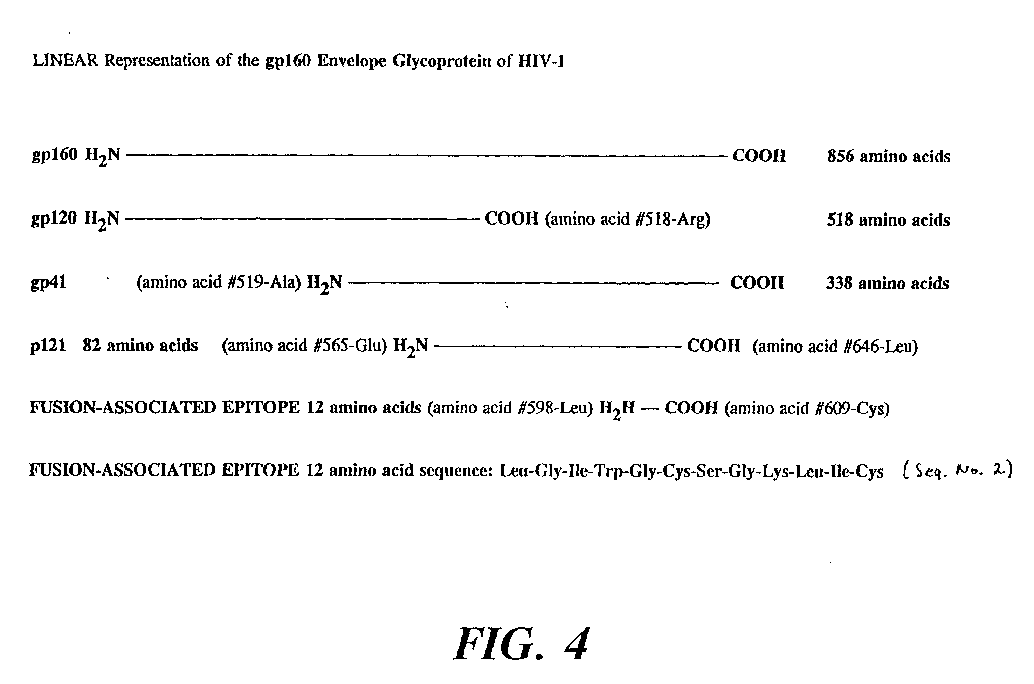

Supernatants from culture wells demonstrating cell growth were assayed for the presence of human anti-HIV antibodies using commercially prepared ELISA plates coated with whole-viral (LAV = lymphadenopathy virus) lysates (e.g., those produced by Genetic Systems, Seattle, WA) and envelope glycoprotein, specifically gp160 (e.g., those produced by MicroGeneSys, West Haven, CT) as target antigens. The human monoclonal antibody (produced by Clone 3, ATCC No. ATCC CRL 10198) reacts with the envelope (env) gene encoded protein gp160 in ELISA testing, and more specifically gp41, the transmembrane glycoprotein envelope component and is designated Anti-gp41. The specificity was determined using the recombinant peptide 121 (p121) as the target antigen in ELISA testing. The p121 is a polypeptide that contains about one-half of the gp41 sequence (the amino terminal half), a major portion of the immunodominant epitopes. (Chang, et al., Bio/Technology (1985) 3:905-999). The human monoclonal antibody does not react with gp120 when tested in a parallel ELISA method against the (external) surface envelope glycoprotein. The specificity results are set forth in Table 1 below. FIGURE 4 depicts a linear comparison of the peptide sequences tested.

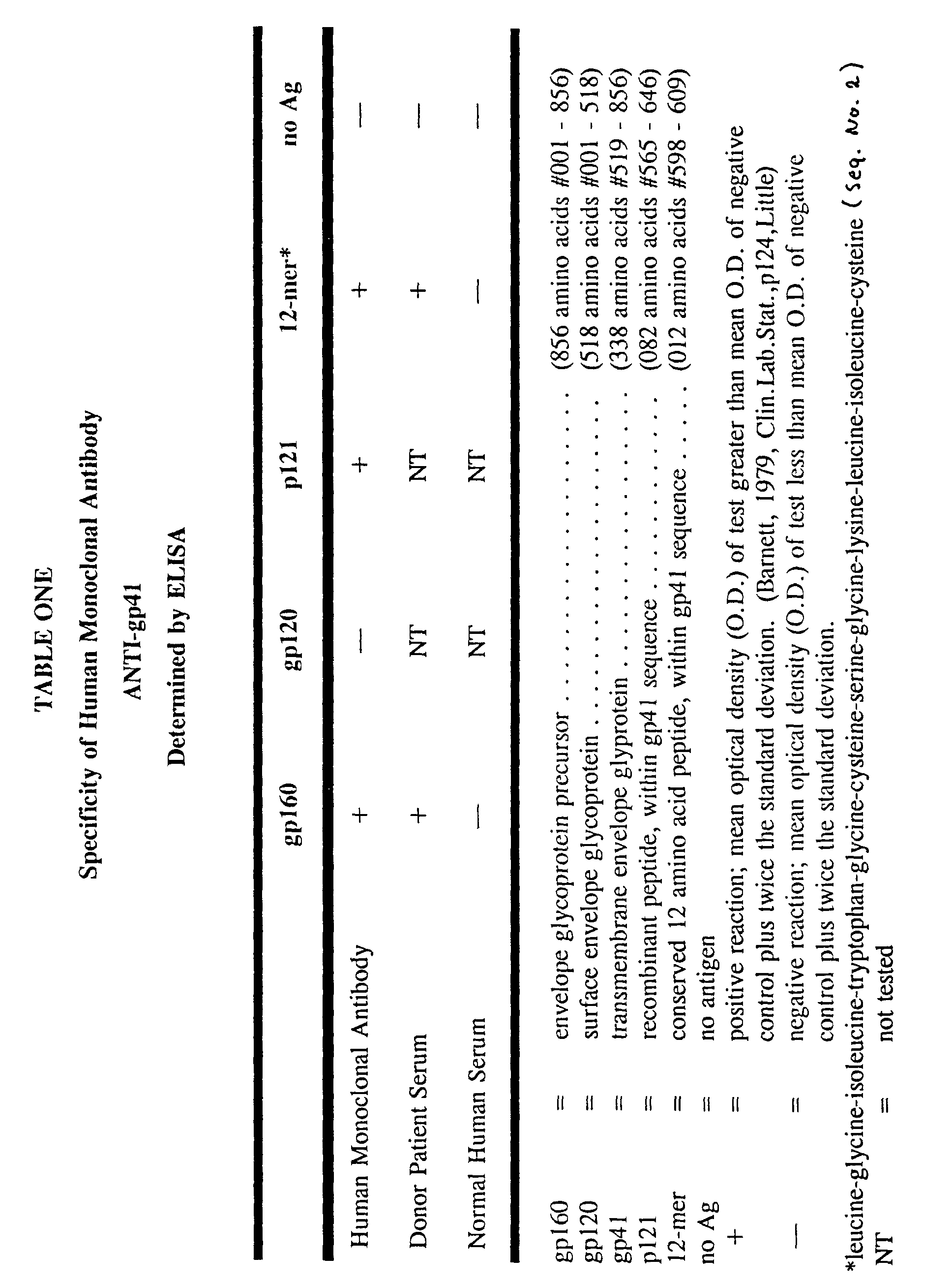

### Example 3

### Immunoblotting

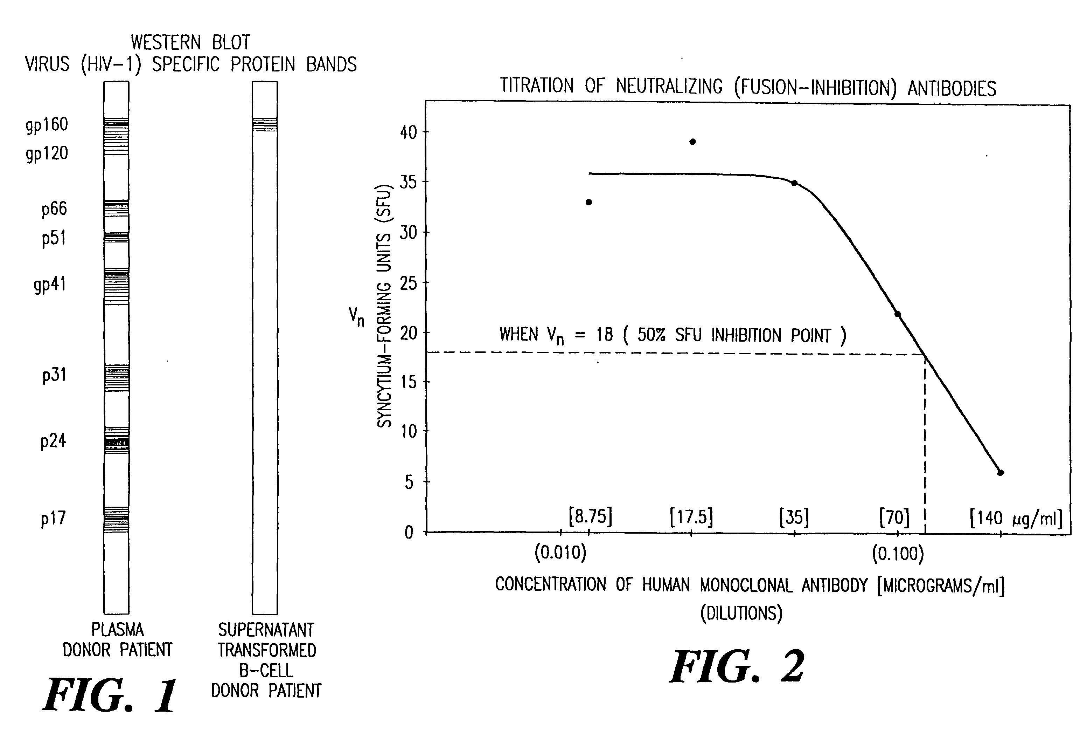

Additional analyses of specificity were carried out by Western blot (WB) with Biotech Research Labs (Rockville, MD) and Immunetics (Cambridge, MA) HIV-antigen preblotted nitrocellulose membranes using standard techniques. The human monoclonal antibody reacts with an HIV-1 major antigen component of 160 kilodaltons (kDa) in viral lysates. It has been determined that noncovalently associated tetramers of gp41 represent the native form of the transmembrane glycoprotein in virions, and that monoclonal antibodies preferentially recognize the oligomeric complexes over monomeric gp41 in Western blots (Pinter, et al., J. Virol. (1989) 63:2674-2679). FIGURE 1 depicts the Western blot testing of the donor patient plasma and the supernatant from the transformed human B cell line using Biotech Research Labs HIV-antigen preblotted nitrocellulose membranes. The results demonstrate a positive reaction for the supernatant corresponding to a band at 160 kDa and co-migrating with gp160.

### Example 4

### Immunoblotting Under Reducing Conditions

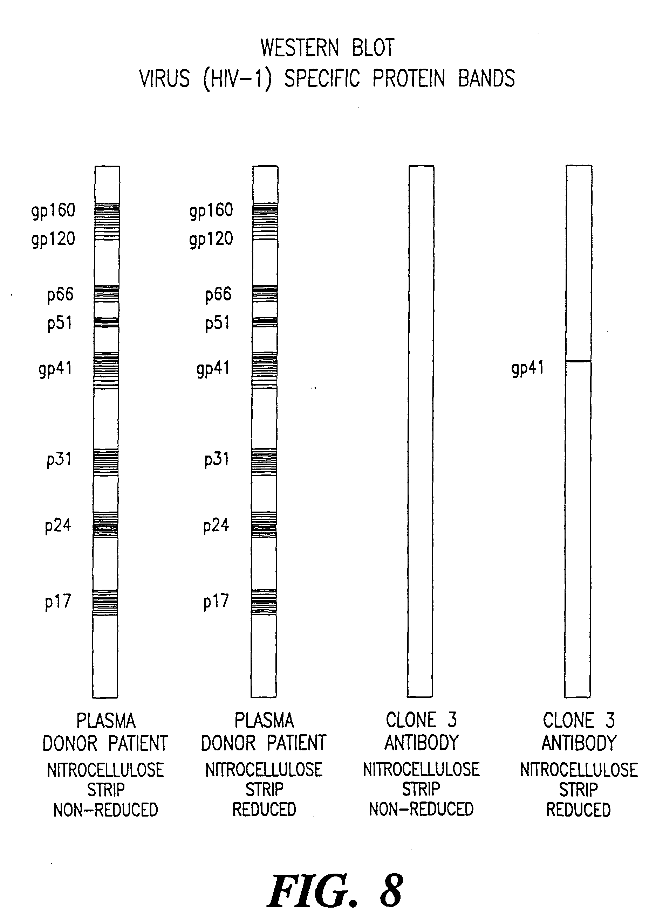

FIGURE 8 represents the Western blot (WB) testing of the donor patient plasma and the affinity-chromatography purified IgG human monoclonal Anti-gp41 antibody (Clone 3 Antibody) using Immunetics (Cambridge, MA) HIV-antigen preblotted nitrocellulose membranes.

In order to reduce disulfide bridges (both intermolecular and intramolecular) that might have formed due to the two invariant cysteine residues, contained within the gp41, mediating the formation of oligomers or cyclic antigenic conformations (reference: Berman, P.W., et al., Journal of Virology, August 1989 63:(8), pp. 3489-3498, Expression and Immunogenicity of the Extracellular Domain of the Human Immunodeficiency Virus Type 1 Envelope Glycoprotein, gp160) the commercially obtained (Immunetics) HIV-antigen preblotted nitrocellulose strips were prepared, immediately prior to utilization, with the following modification of the standard procedure described below:

The HIV-antigen preblotted nitrocellulose strips were incubated in PBS buffer, pH 7.4, with β-mercaptoethanol (1% volume/volume), a reducing agent, at either 37°C. for two hours or 95°C for two minutes.

Standard immunoblotting technique was then carried out for testing of the samples described above, utilizing both reduced and non-reduced preblotted nitrocellulose strips, in a parallel procedure.

The significant results demonstrated a (weak) positive reaction for the human monoclonal antibody (Clone 3 Antibody) corresponding to a band at 41 kDa and co-migrating with gp41 only when the HIV-antigen preblotted nitrocellulose strip has been reacted with the reducing agent β-mercaptoethanol. No corresponding reaction band was noted between the human monoclonal antibody (Clone 3 Antibody) and the HIV-antigen migrating at 41 kDa when the HIV antigen preblotted nitrocellulose strip was not subjected to pretesting reduction treatment with β-mercaptoethanol.

These data indicate that the human monoclonal antibody (Clone 3 Antibody) reacted preferentially with the reduced (linear) gp41 fusion-associated octapeptide epitope, GCSGKLIC (Seq. No. 1), in immunoblotting.

### Example 5

### Affinity Chromatography Isolation and Purification of Human Monoclonal IqG

Aliquots (500 ml volumes) of hybridoma cell culture supernatants (spent cell culture media) were subjected to batch immunoadsorption utilizing Sepharose® 4-Fast Flow coupled protein G (Pharmacia, Piscataway, NJ), a recombinant streptococcal IgG Fc receptor, that has the capacity of adsorbing 17 mg of human IgG (all subclasses) per ml of gel. The immunoadsorbed monoclonal antibody was eluted from the matrix via low pH buffer (0.1 M glycine-HCl, pH 2.4) with rapid neutralization of eluate by collection into 1.0 M TRIS-HCl buffer (pH 9.0) for a final pH of 7.8.

### Example 6

### Determination of Immunoglobulin Class and Subclass

The class and light-chain type of Anti-gp41 monoclonal antibody were determined by ELISA. For these assays, commercially-prepared microtiter plates coated with gp160 (MicroGeneSys) were incubated with culture supernatants (or purified human monoclonal anti-gp41 IgG). The type (class) of antibody was determined with the following horseradish peroxidase-coupled antibodies: goat anti-human IgG (γ chain specific) and goat anti-human IgM (*µ* chain specific) (Zymed Laboratories, San Francisco, CA). The subclass of the human monoclonal antibody was also analyzed by ELISA with horseradish peroxidase conjugated mouse monoclonal antibodies against the four subclasses (IgG1, IgG2, IgG3, IgG4) of human IgG (Zymed Laboratories, San Francisco, CA). The light-chain type of Anti-gp41 was determined by exclusion using peroxidase-labeled mouse monoclonal antibody anti-human κ chain. The immunological characterization data of the human monoclonal antibody (Anti-gp41) indicated that the immunoglobulin was of the IgG class, specifically subclass 1, with non-κ, therefore probably λ, light-chain determinants.

In an additional immunologic study, in a dot-immunobinding assay (DIBA) on nitrocellulose membranes, as described by Jol-Van der Zijde, et al., Journal of Immunological Methods (1988) 108:195-203, the human monoclonal antibody (Clone 3 Antibody) was demonstrated to react with antiserum (Zymed Laboratories, San Francisco, CA) monospecifically directed against the λ light-chain.

### Example 7

### Quantitation of Anti-gp41 Human Monoclonal IgG1

IgG quantitation was performed on affinity purified protein by the Lowry technique using purified protein (albumin) to produce the standard curves used in calculations. (Lowry, et al., J. Biol. Chem. (1951) 193:265). The concentration of IgG produced by the cell lines varied, but generally reached a maximum at day 9 of culture, ranging from 2-10 micrograms/ml.

### Example 8

### Epitope Mapping of Human IgG1 Monoclonal Anti-gp41 Antibody Binding to Transmembrane Glycoprotein gp41

The wells of Immulon II microELISA plates (Dynatech Industries, McLean, VA) were coated for a minimum of 12 hours at 4°C with a solution of the synthetic peptide 2, leucine-glycine-isoleucine-tryptophan-glycine-cysteine-serine-glycine-lysine-leucine-isoleucine-cysteine (Seq. No. 2) (Cambridge Research Biochemicals, Valley Stream, NY). The peptide was solubilized initially with 10% acetic acid and then brought to a final concentration of 10 *µ*g/ml, pH 6.4 in PBS. One hundred microliters of this solution was added per well. In order to minimize polymerization, β-mercaptoethanol (0.5% vol/vol) was added to the solution. The wells were emptied of peptide solution, washed thrice with 300 microliters PBS containing 0.05% polyoxyethylene (20) sorbitan monolaurate (e.g., Tween 20, produced by ICI Specialty Chemicals) then twice with 300 microliters of PBS alone. Excess binding sites were blocked (quenched) with 200 microliters of PBS containing 1% bovine serum albumin (BSA) per well. Wells containing no peptide were subjected to the blocking process to be utilized as one parameter of negative controls. The wells were incubated with the blocking solution for 3 hours at room temperature, then emptied by aspiration, and washed thrice with 300 microliters of PBS. The wells were emptied, dried and the peptide-coated plates were then stored with desiccant in a sealed bag at 4°C. The peptide-coated plates are stable for at least 3 months when stored in this manner.

To test the binding characteristics of the human monoclonal Anti-gp41 antibody with the synthetic peptide, affinity-chromatography purified IgG (700 micrograms/ml) was serially diluted in PBS with 0.05% polyoxyethylene (20) sorbitan monolaurate and 1% BSA and reacted (100 microliters) in the peptide-coated wells and in wells without antigen (negative control) for 2 hours at 37°C. Normal human serum (negative control) and the donor patient serum were diluted 1:401 and reacted in the ELISA test against the peptide-coated wells and in wells without antigen (negative control). After the initial incubation period, the test samples and controls were aspirated from the wells which were then washed 9 times with 300 microliters of 0.05% polyoxyethylene (20) sorbitan monolaurate in PBS. A solution of 100 microliters of goat anti-human IgG conjugated to horseradish peroxidase was diluted 1:100 and added to the wells. After 2 hours at 37°C, the wells were washed again with 0.05% polyoxyethylene (20) sorbitan monolaurate in PBS, 9 times, and then incubated with 100 microliters of tetramethylbenzidine chromogen in dimethylsulfoxide (DMSO) with buffered hydrogen peroxide substrate for 30 minutes at room temperature. The reaction was stopped by the addition of 100 microliters of 1N sulfuric acid (H₂SO₄) and the optical density or absorbance of the solution determined at 490 nm, a colorimetric determination in the visible spectrum.

The results indicate that the human monoclonal antibody binds specifically to the gp41 peptide with the amino acid sequence leucine-glycine-isoleucine-tryptophan-glycine-cysteine-serine-glycine-lysine-leucine-isoleucine-cysteine (Sep. No. 2), as did the patient serum. Normal human serum controls were negative. Additionally, the human monoclonal antibody, patient serum, and normal human serum did not bind to uncoated (no antigen) wells of the Immulon II ELISA plate. (See Table 1).

### Example 9

### Epitope Mapping of Human IqG1 Monoclonal Anti-gp41 Antibody Binding to Transmembrane Glycoprotein gp41

The biochemically and immunologically defined human monoclonal antibody is produced by the Clone 3 cell line (ATCC CRL 10198) and is directed against the HIV-1 transmembrane glycoprotein gp41, specifically the 12 amino acid peptide (12-mer = Peptide 2), Leu-Gly-Ile-Trp-Gly-Cys-Ser-Gly-Lys-Leu-Ile-Cys (Seq. No. 2) (Example 8, Table 1), which has two potential antibody binding regions (epitopes).

Others (Mathiesen, et al., Immunology (1989) 67:1-7) have recently investigated the **binding capacity only of polyclonal** human IgG antibody with two overlapping HIV-1 gp41 peptides (E34/E32; amino acid positions 587-608 and 600-618) in order to define the amino acids involved in epitopes and antibodies interactions.

The IgG **paratopes** showed reactivity to two regions (Mathiesen, id.: page 3, first column; page 4, Table 3) within the Peptide 2 sequence, Leu-Gly-Ile-Trp-Gly-Cys-Ser-Gly-Lys-Leu-Ile-Cys (Seq. No. 2), under consideration in this patent. The **two paratopes** reacted with the regions within the Peptide 2 sequence consisting of the amino acid sequences **Ile-Trp-Gly** and **Gly-Cys-Ser-Gly-Lys-Leu-Ile-Cys** (Seq. No. 2).

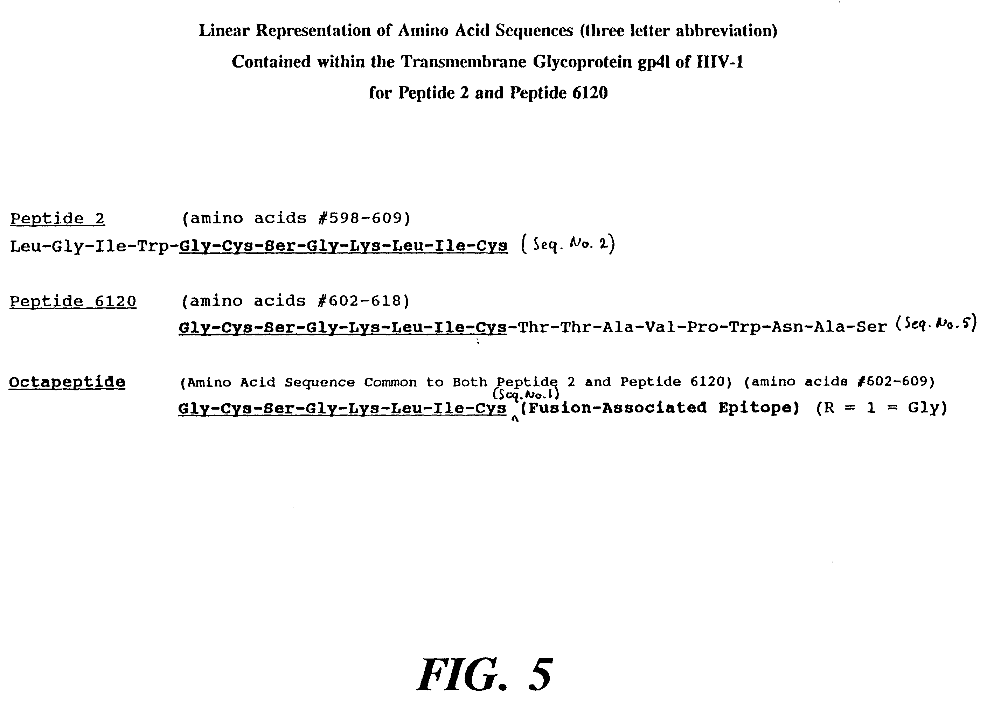

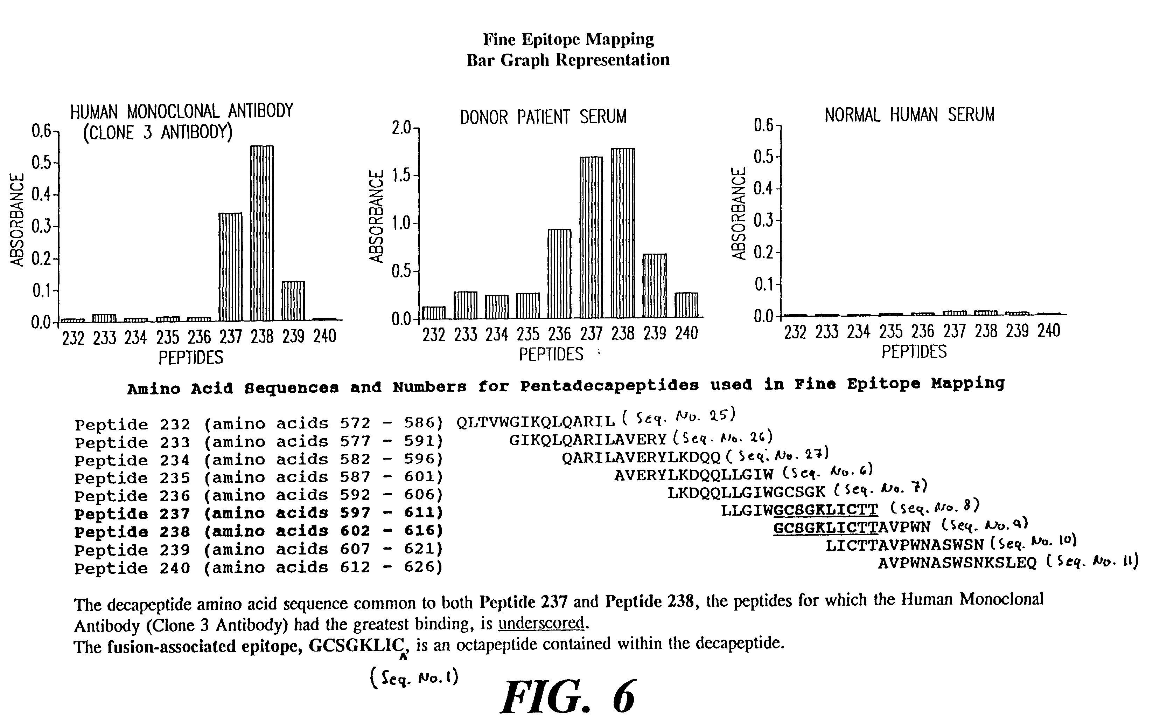

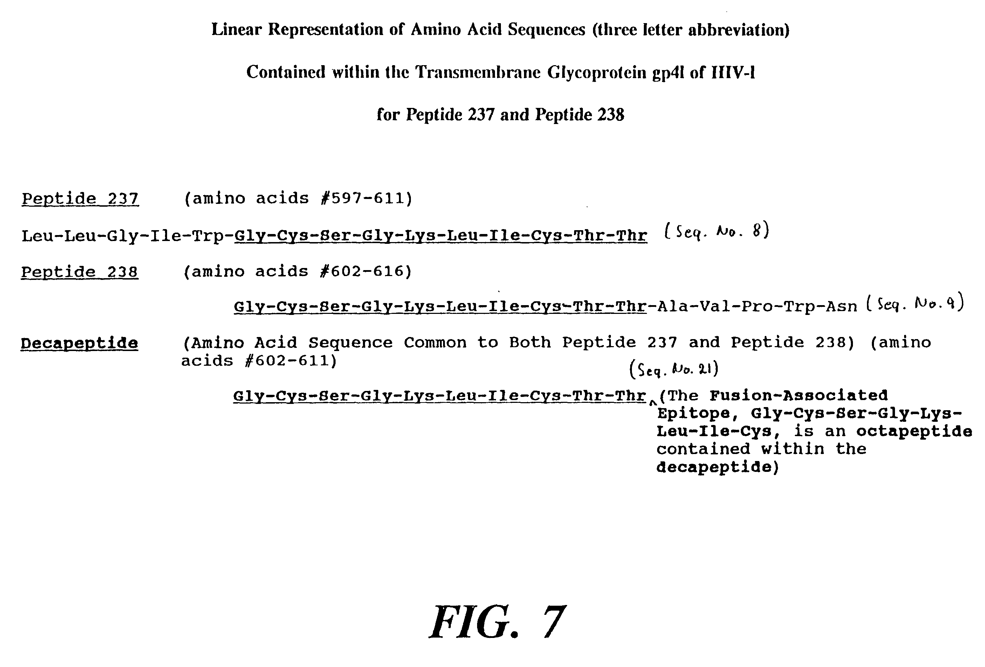

As shown below in Example 9, Table Two, FIGURE 5; Example 10, Table Three, FIGURE 6 and FIGURE 7, the human monoclonal antibody (Clone 3 antibody) binds preferentially to at least a portion of the amino acid sequence Gly-Cys-Ser-Gly-Lys-Leu-Ile-Cys (Seq. No. 1), an octapeptide (8-mer) which contains only one complete epitope of the two antibody binding regions within the 12-mer.

The interaction of the fusion blocking human monoclonal antibody with the identified structural octapeptide sequence (8-mer) (Example 9, Table Two, FIGURE 5) thereby ascribes for the first time an associated neutralizable physiological function to the immunogenic octapeptide epitope, that being fusion-associated function.

The biological function of the delineated **single** epitope, consisting of the octapeptide amino acid sequence **Gly-Cys-Ser-Gly-Lys-Leu-Ile-Cys (8-mer)** (seq. No. 1), denoted as **fusion-associated epitope,** is not known to have been described previously in any published or presented scientific paper. These data are presented in Examples 11 and 18 herein.

The separate wells of Immulon II microELISA plates (Dynatech Industries, McLean, VA) were coated for a minimum of 12 hours at 4°C with a solution of the synthetic cyclic peptide 6120 or linear peptide glycine-cysteine-serine-glycine-lysine-leucine-isoleucine-cysteine-threonine-threonine-alanine-valine-proline-tryptophan-asparagine-alanine-serine (Seq. No. 5). The cyclic peptide 6120 (IAF BioChem International, Inc., Montreal, Canada) was solubilized initially with 10% acetic acid and then brought to a final concentration of 10 micrograms/ml, pH 7.4 in PBS. In order to minimize polymerization and cyclization and to reduce the cyclic peptide, β-mercaptoethanol (1% vol/vol) was incubated with an aliquot solution (Ph 6.4) for 1 hour at 37°C. Then, one hundred microliters of this reduced, linear peptide solution was added per well. The wells were emptied of peptide solution, washed thrice with 300 microliters PBS containing 0.05% polyoxyethylene (20) sorbitan monolaurate (e.g., Tween 20, produced by ICI Specialty Chemicals) then twice with 300 microliters of PBS alone. Excess binding sites were blocked (quenched) with 200 microliters of PBS containing 1% bovine serum albumin (BSA) per well. Wells containing no peptide were subjected to the blocking process to be utilized as one parameter of negative controls. The wells were incubated with the blocking solution for 3 hours at room temperature, then emptied by aspiration, and washed thrice with 300 microliters of PBS. The wells were emptied, dried and the peptide-coated plates were then stored with desiccant in a sealed bag at 4°C. The peptide-coated plates were stable for at least 3 months when stored in this manner.

To test the binding characteristics of the human monoclonal Anti-gp41 antibody with the synthetic peptide 6120, affinity-chromatography purified IgG, (1 mg/ml) was serially diluted in PBS with 0.05% polyoxyethylene (20) sorbitan monolaurate and 1% BSA and reacted (100 microliters) in the peptide-coated wells and in wells without antigen (negative control) for 2 hours at 37°C. Normal human serum (negative control) and the donor patient serum were diluted 1:401 and reacted in the ELISA test against the peptide-coated wells and in wells without antigen (negative control). After the initial incubation period, the test samples and controls were aspirated from the wells which were then washed 9 times with 300 microliters of 0.05% polyoxyethylene (20) sorbitan monolaurate in PBS. A solution of 100 microliters of goat anti-human IgG conjugated to horseradish peroxidase was diluted 1:100 and added to the wells. After 2 hours at 37°C, the wells were washed again with 0.05% polyoxyethylene (20) sorbitan monolaurate in PBS, 9 times, and then incubated with 100 microliters of tetramethylbenzidine chromogen in dimethylsulfoxide (DMSO) with buffered hydrogen peroxide substrate for 30 minutes at room temperature. The reaction was stopped by the addition of 100 microliters of 1N sulfuric acid (H₂SO₄) and the optical density or absorbance of the solution determined at 490 nm, a colorimetric determination in the visible spectrum.

The results indicate that the human monoclonal antibody (Clone 3 Antibody) binds specifically to the gp41 peptide with the amino acid sequence glycine-cysteineserine-glycine-lysine-leucine-isoleucine-cysteine-threonine-threonine-alanine-valine-proline-tryptophan-asparagine-alanine-serine (Seq. No. 5) (reduced, linear, and cyclic), as did the donor patient serum. Normal human serum controls were negative. (Table Two).

The amino acid sequence common to both Peptide 2 (12-mer) and Peptide 6120 **and** to which Clone 3 human monoclonal antibody binds is the octapeptide (8-mer) glycine-cysteine-serine-glycine-lysine-leucine-isoleucine-cysteine (Seq. No. 1). (See FIGURE 5).

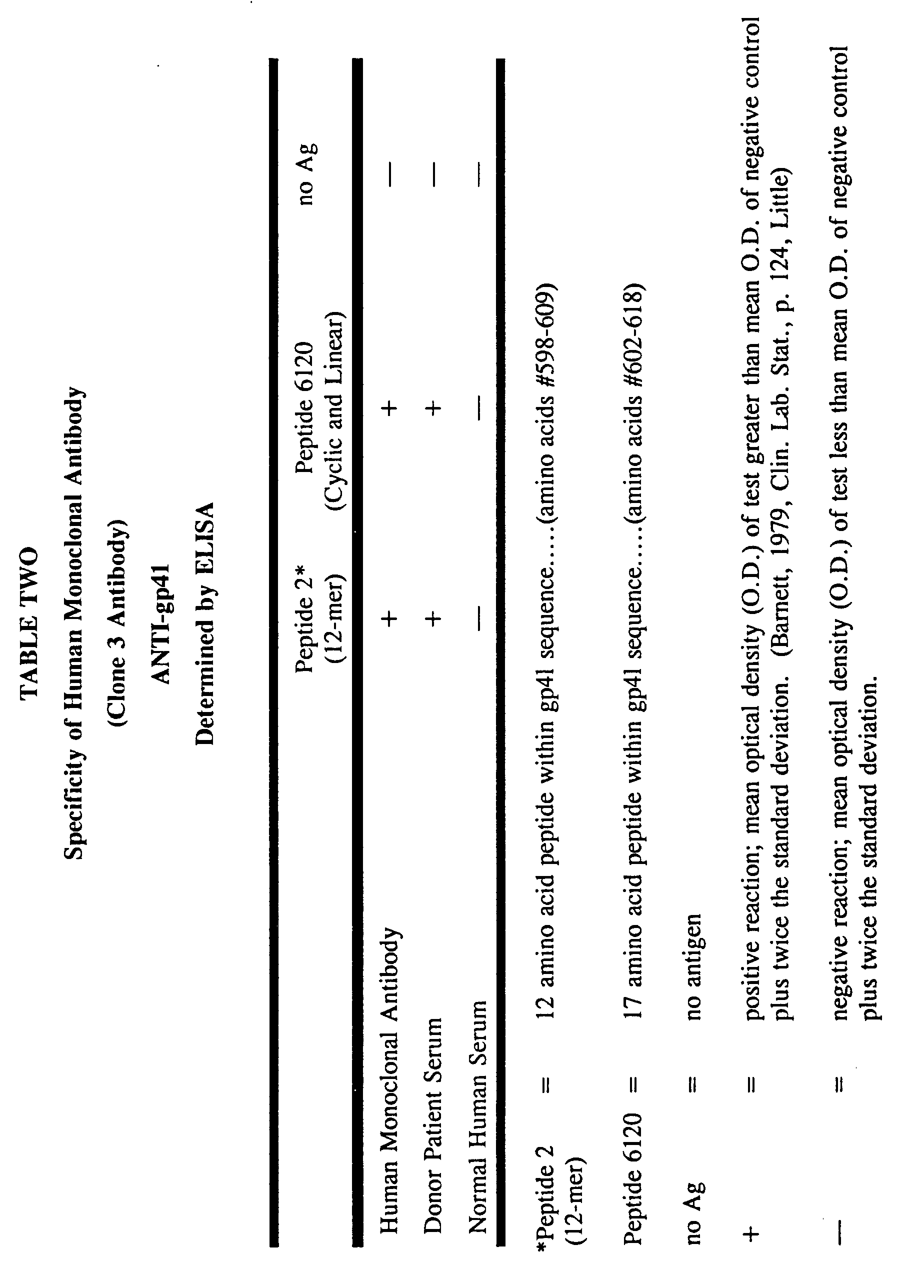

### Example 10

### Fine Epitope Mapping of Human IgG1 Monoclonal Anti-gp41 Antibody by ELISA

### Using Synthetic Pentadecapeptides (15 Amino Acid Peptides)

Pentadecapeptides, sequentially overlapping by 10 amino acids were synthesized on the basis of HTLV-IIIB, clone B10 (Ratner et al., Nature (1985) 313:277), for gp41 peptides 232 - 240 (amino acids 572 - 626). These were synthesized according to the solid-phase method of Merrifield, J. Amer. Chem. Soc. (1963) 85:2149, modified by Houghten, Proc. Natl. Acad. Sci. U.S.A. (1985) 82:5131. For human monoclonal antibody ELISAs, microwell plates (Nunc Immunoplate I) were coated with 1 microgram of peptide/microwell. Sera samples and affinity column (Protein G) purified human monoclonal antibody were assayed in dilutions of 1:50 for 105 minutes at 37°C. Mouse antibodies to IgG were used to bind to human IgG and subsequently detected by HRPO-conjugated anti-mouse Ig. A color reaction was obtained with ortho-phenylenediamine (OPD) and the optical density (0D) at 490 nm was recorded. Blanks and HIV seronegative controls were included in all plates. Sera and human monoclonal antibody giving greater than 3 times the mean 0D of negative controls (always more than mean + 3SD) were scored as positive for IgG and peptide.

The results indicate that the human monoclonal antibody (Clone 3 Antibody) binds specifically to **Peptide 237** and **Peptide 238,** as did the donor patient serum. Normal human serum controls were negative. (Table Three and FIGURE 6). The **decapeptide** sequence common to both Peptide 237 and Peptide 238 contains the **octapeptide** amino acid sequence, the **fusion-associated epitope** (FIGURE 7).

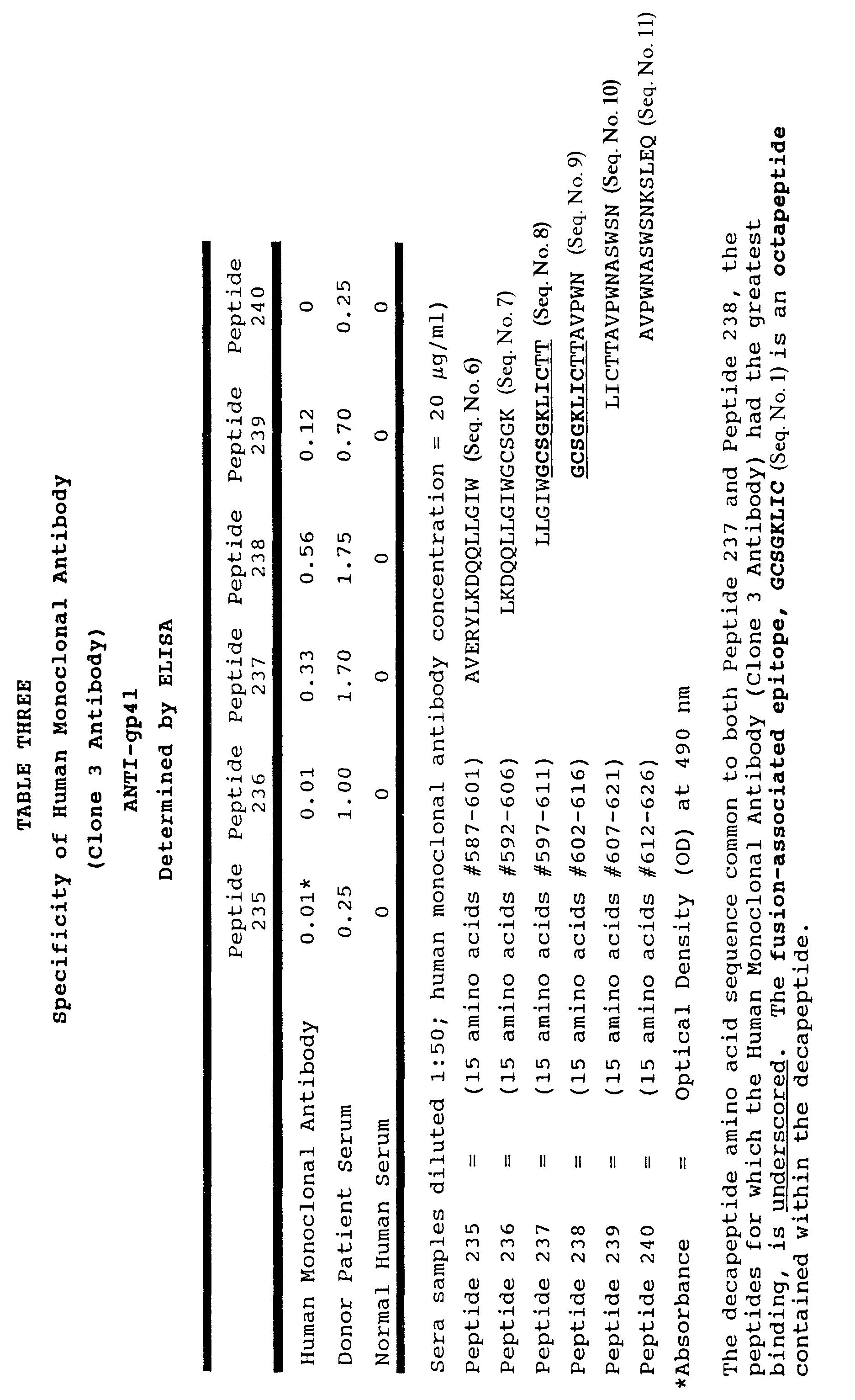

### Example 11

### Comparison of Immunoreactivity for Peptides Used in Fine Epitope Mapping of Clone 3 Antibody

As is noted in the application and disclosed in the specification in Table Three, page 40, and FIG. 6, Clone 3 Antibody immunoreactivity (ELISA) tested positive for Peptides 237, 238 and 239. As noted in Table Two, page 37, Peptide 2 (linear) and Peptide 6120 (cyclic and linear) demonstrated a positive Clone 3 Antibody immunoreactivity (ELISA) as well. However, Peptides 236 and 240 showed a negative Clone 3 Antibody immunoreactivity (ELISA). (See FIG. 6 of the application.) These data can be summarized using the following chart which compares the immunoreactivity for the peptides used in fine epitope mapping of Clone 3 Antibody.

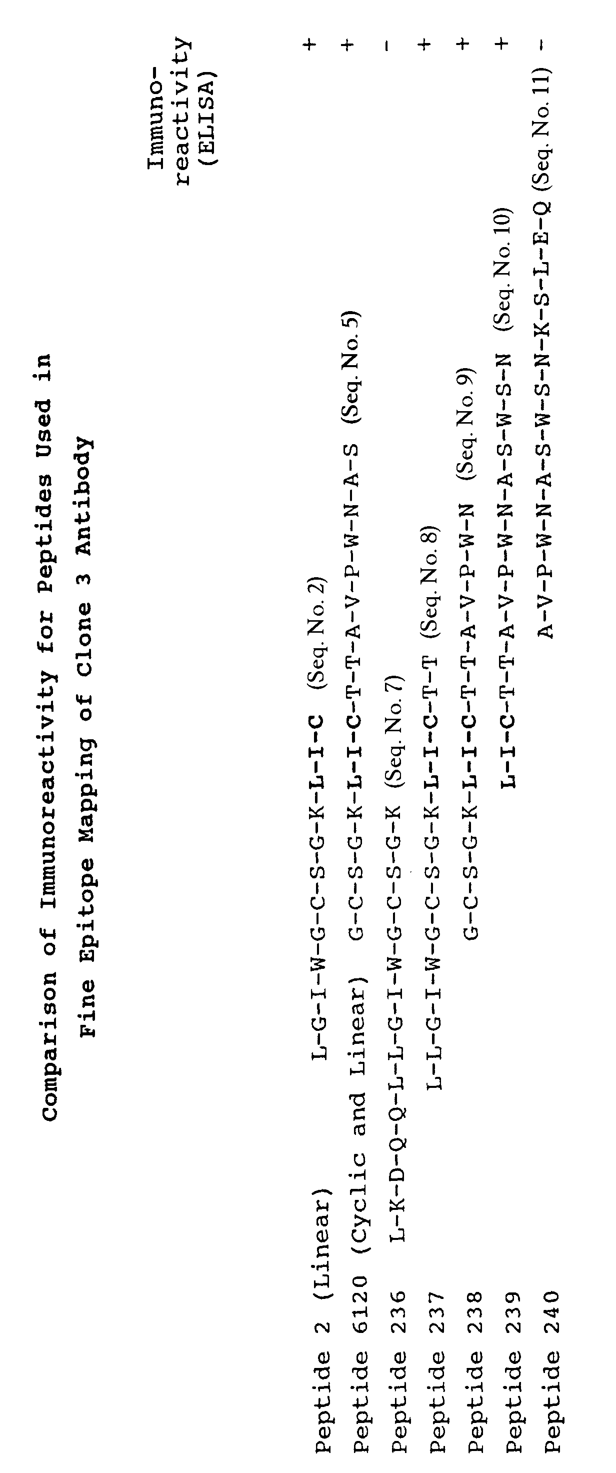

The amino acid sequence consisting of leucine-isoleucine-cysteine (**L-I-C**)is present in those peptides to which Clone 3 Antibody binds (displays a positive immunoreactivity) and are not present in Peptide 236 and Peptide 240 to which Clone 3 antibody does not bind (displays no immunoreactivity). These results indicate that the minimal amino acid sequence within the defined epitope GCSGKLIC to which Clone 3 Antibody specifically binds is leucine-isoleucine-cysteine (Seq. No. 7) (**L-I-C**).

### Example 12

### Quantitative Syncytium-Forming Microassay for the Detection of Human Immunodeficiency Virus Neutralizing Antibody

The biological reactivity of the human monoclonal antibody (Clone 3 Antibody) has been demonstrated in inhibition of syncytia formation assays wherein the neutralization capacity of the human monoclonal antibody is characterized by the blocking of fusion between HIV-1 infected and uninfected human cells (Example 11, Table Four, FIGURE 2) and by neutralization of free HIV-1 (SF2) infectivity as presented below in Example 18 and Table Five.

Traditionally, retroviruses, including HIV-1, can be assayed directly by a number of simple focus-forming or syncytium-forming assays. (Nara, et al., AIDS Research and Human Retroviruses (1987) 3:283-302; Putney, et al., Science (1986) 234:1392-1395). These assays are very sensitive, simple, relatively rapid, and allow for ready biological assessment concerning infectivity of virus under various in vitro conditions. Additionally, various virus-envelope associated properties such as interference by inhibitory agents or neutralization by antibodies can be studied.

The human monoclonal antibody anti-gp41 was tested in the syncytium-forming microassay procedure, as described by Putney, to ascertain HIV-1 neutralization capabilities as demonstrated by fusion inhibition. The microtiter syncytium-forming assay utilized a clone of CEM (CD4+) cells chronically infected with the HIV-1 isolate HTLV-IIIB, and MOLT-4 (CD4+) cells. CEM cell stocks stably infected with HTLV-IIIB, yet not susceptible to the cytopathological effects of the virus, were used as the infected partner and MOLT-4 cells were used as the uninfected partner. Cells were washed once in growth media and cell concentrations were adjusted to 0.125 x 10⁶ cells/ml and 1.75 x 10⁶ cells/ml for CEM and MOLT-4 cells, respectively. Ninety-six well, half-area plates were used in the assay. Anti-gp41 antibody solution was added to half-area wells in a volume of 10-20 microliters. Forty microliters of each cell solution was then added to the well, resulting in a ratio of approximately 5,000 HTLV-IIIB-infected CEM cells to 70,000 uninfected MOLT-4 cells per well. Plates were incubated at 37°C in an atmosphere of 5% CO₂ for 20-24 hours.

Syncytia formation of cells, or giant cell formation, was enumerated using an inverted microscope at 40X magnification. The number of giant cells, defined as multinucleated fused cells being 5 times the diameter of input cells, were scored as the number of syncytium-forming units per well.

The results of fusion inhibition by Anti-gp41 are quantitatively reported. The human monoclonal antibody concentration with the corresponding syncytium-forming units (SFUs) observed are presented in Table Four below, where Vₒ is the total number of virus induced SFUs per well in the absence of antibody and Vₙ is the number of SFUs per well in the presence of antibody, in doubling dilutions of (decreasing concentration).

**TABLE FOUR**

| **Syncytium-Forming Microassay** | | |
|---|---|---|
| **HUMAN** **Monoclonal Antibody* Dilution** | | **Number of Syncytium- Forming Units (SFU) V**_{**n**} |
| 1:5 | (140 *µ*g/ml) | 6 |
| 1:10 | (70 *µ*g/ml) | 22 |
| 1:20 | (35 *µ*g/ml) | 35 |
| 1:40 | (17.5 *µ*g/ml) | 39 |
| 1:80 | (8.75 *µ* g/ml) | 33 |
| Control (no antibody) (Vₒ) | | 36 |

| | | |
|---|---|---|
| SFU average of replicates of quadruplicate determinations (Vₙ and Vₒ) *700 micrograms/ml = (protein concentration neat) | | |

The human monoclonal Anti-gp41 antibody at a 1:5 dilution (the minimum dilution allowed and therefore the maximum antibody concentration tested in the microassay system) decreased in number the formation of syncytium-forming units between HIV-1 (HTLV-IIIB) infected CD4+ cells and uninfected target CD4+ (MOLT-4) cells from 36 (Vₒ) to 6 (Vₙ) for a fusion inhibition of 83%. Replicate tests were performed in quadruplicates, and fusion inhibition percentage values were calculated from the reduction in virus-induced, syncytial-forming units, represented as (Vₒ-Vₙ), obtained in the presence of twofold dilutions of human monoclonal Anti-gp41, divided by the number of total virus induced SFUs added (Vₒ).

In the microassay system, the concentration of the affinity column (Protein G) purified human monoclonal Anti-gp41 IgG1 that resulted in an 83% inhibition in syncytium-formation was 140 micrograms/ml, a physiological concentration. The 50% SFU inhibition point (Vn=18) was obtained at a human monoclonal Anti-gp41 IgG1 antibody concentration of -88 micrograms/ml. (See FIGURE 2).

### Example 13

### Therapeutic Uses For Anti-gp41

The antibody of the invention may be used therapeutically, as described below, in the modality of passive immunotherapy. Human monoclonal antibodies with the proper biological properties are useful directly as therapeutic agents. Data to support the efficacy and delineation of the therapeutic protocols for passive immunotherapy in other primates (chimpanzees) have been published, for it has been determined that neutralization of in vivo HIV-1 infectivity can be mediated by in vitro neutralizing antibody directed against the hypervariable loop of the viral envelope (gp120). Chimpanzee-derived polyclonal antibodies were utilized in the protocol. (Emini, et al., V. International Conference on AIDS (1989), Abstract No. Th.C.O.30, p. 538). Emini, E.A., et al., J. Virol., August 1990, 64:3674-3678, Antibody-Mediated In Vitro Neutralization of Human Chimpanzees; Emini, et al., V. International Conference on AIDS (1989), Abstract No. Th.C.O.30, p. 538. By administering an appropriate human monoclonal antibody to patients who lack neutralizing antibodies against the envelope epitope within gp41, passive immunotherapy can be provided.

### PROCEDURE AND DATA OF EXPERIMENTAL PROTOCOL DEMONSTRATING EFFICACY FOR PASSIVE IMMUNOTHERAPY IN VIVO

With regard to protective passive immunization in vivo, primate research protocols have established that both chimpanzee polyclonal and xenogeneic (mouse) monoclonal antibody, directed against the human immunodeficiency virus type 1 (HIV-1) gp120 V3 loop, can abolish or delay the infectivity of HIV-1 in chimpanzees, respectively.

Virus and antibody specific for the gp120 V3 loop were mixed in vitro under conditions leading to complete in vitro neutralization of the virus. The virus-antibody mixture was then inoculated into a susceptible chimpanzee. Including controls, four purified immunoglobulin G (IgG) samples were tested: (i) **nonneutralizing** control chimpanzee IgG, (ii) **nonneutralizing** anti-HIV-1 polyclonal human IgG, (iii) a **neutralizing** murine monoclonal antibody (0.5β) against the gp120 V3 loop, and (iv) a **neutralizing** polyclonal chimpanzee IgG directed against the gp120 V3 loop.

### Materials and Methods:

Challenge inocula of the HTLV-IIIb (HIV-1) isolate (140 chimpanzee 50% infectious doses per ml) were mixed in vitro with each protein A-affinity chromatography purified antibody preparation (1 ml) and then incubated at room temperature (19°C to 25°C) for 90 minutes. The two virus-neutralizing antibody preparations were mixed with the virus inoculum in excess of the minimum levels needed for complete virus neutralization, as determined by in vitro virus neutralization assays. Each mixture was then inoculated intravenously into one of four HIV-seronegative chimpanzees. The animals were subsequently tested for HIV-1 infection by virus isolation. Isolation results were confirmed by polymerase chain reaction (PCR)-mediated amplification of virus-specific DNA sequences from peripheral blood mononuclear cells (PBMC). The development of specific antibody responses against HIV-1 antigens, an indication of persistent infection, was also assessed.

After incubation of virus with antibody, an aliquot (1/40th of each mixture) was withdrawn and analyzed for its in vitro infectivity with the highly HIV-susceptible MT-4 lymphoid cell line. The virus inocula mixed with the nonneutralizing IgG preparations (i) and (ii) both proved infectious, yielding virus-specific immunofluorescence several days after inoculation onto MT-4 cells. In contrast, no virus-specific infection was observed for 8 weeks following cell culture inoculation of virus mixed with neutralizing IgG preparations (iii) and (iv).

The remainder of each virus-antibody mixture was injected intravenously into one of four HIV-seronegative chimpanzees. Each animal was then assessed for the development of persistent HIV-1 infection.

The animal inoculated with the virus-preparation (i) control chimpanzee IgG mixture exhibited initial immunological signs of HIV-1 infection by 6 weeks post-inoculation (p.i.), as demonstrated by enzyme-linked immunosorbent assay (ELISA) and Western blot analysis. Neutralizing antibody to the challenge inoculum developed at 10 weeks p.i., and all virus isolation attempts from PBMC yielded virus. Similar observations were made with the animal which had received the virus-nonneutralizing anti-HIV-1 polyclonal seropositive human preparation (ii) IgG mixture.

In contrast, the chimpanzee inoculated with the virus-**neutralizing** chimpanzee polyclonal preparation (iv) IgG mixture, did not develop serological signs of HIV-1 infection throughout the entire 76-week observation period. Additionally, HIV-1 virus was not isolated at any time. The chimpanzee inoculated with the virus-neutralizing murine monoclonal preparation (iii) IgG mixture, exhibited a delayed serological response to the virus. Specific virus Western blot-reactive and virus ELISA-reactive antibodies were first noted at 12 and 14 weeks p.i., respectively. The ELISA titers remained low through 31 weeks of observation and the Western blot reactivity patterns were restricted to four or fewer viral proteins during this time. Neutralizing antibody to the challenge inoculum was not noted until the week 67 sample. The ability to isolate virus from the PBMC was sporadic.

Additionally, Emini has extended earlier finding in chimpanzees with regard to passive immunization protection against in vivo HIV-1 infection via pre-challenge administration of polyclonal anti-V3 loop antibodies to include such monoclonal antibodies, as reported at the VIIth International Conference on AIDS in Florence, Italy, June 16-21, 1991 (Abstract No. ThA64 page 72). A mouse-human IgG1 chimeric monoclonal antibody specific for the principal neutralization determinant of the HIV-1 IIIb isolate was prepared to passively immunize the chimpanzee, via IV infusion, which was then challenged 24 hours later with 75 chimpanzee ID of the HIV-1 IIIb isolate. A nonimmunized chimpanzee was simultaneously challenged with the virus. The control animal developed immunological signs of infection at 6 weeks postchallenge. The passively immunized chimpanzee has not developed signs of infection up to 18 weeks after challenge.

In a parallel human study, data from recent clinical trials (Jackson, et al., Lancet (1988) 2:647-652; Karpas, A., Proc. Natl. Acad. of Sciences (U.S.A.) (1988) 85:9234-9237) Karpas, A., Proceedings of the National Academy of Sciences (U.S.A.) (1988) 85:9234-9237), Effects of Passive Immunication in Patients with the Acquired Immunodeficiency Syndrome-Related Complex and Acquired Immunodeficiency Syndrome) have demonstrated that passive immunization improved the status of patients with advanced (symptomatic) AIDS. In those trials passive immunization was accomplished by transfusing plasmas containing antibodies from asymptomatic AIDS patients into the symptomatic AIDS recipients.

Karpas has extended and confirmed the earlier findings presenting the data in recent journal publications, (Karpas, A., et al., Proceedings of the National Academy of Science (U.S.A.), (1990), 87:7613-7617, Polymerase Chain Reaction Evidence for Human Immunodeficiency Virus 1 Neutralization by Passive Immunization in Patients with AIDS and AIDS-Related Complex; Karpas, A., et al., Biotherapy, (1990), 2:159-172, Passive Immunization in ARC and AIDS), wherein the studies indicate that passive immunization may be safe in ARC and AIDS patients and that immunotherapy reduces HIV-1 viremia to levels undetectable even by polymerase chain reaction (PCR), and clinically, an initial improvement was noted in all patients.

For example, the passive immunization method against hepatitis B virus has been utilized in humans, when clinically indicated, as routine effective measures of post-viral exposure prophylaxis, wherein hepatitis B immune globulin is administered to the at-risk recipient at a dose of 0.06 ml/kg IM (Center for Disease Control, Department of Health and Human Services. "Recommendations for Protection Against Viral Hepatitis. Recommendation of the Immunization Practices Advisory Committee." MMWR (1990) 39:1-26).

When a neutralizing agent, such as a human monoclonal antibody, is used in passive immunotherapy, the protocol regimen can parallel that delineated by Emini and for the administration of hepatitis B immune globulin (e.g., H-BIG® , a solution of human immunoglobulin obtained from pooled venous plasma of individuals with high titers of antibody to the hepatitis B surface antigen, prepared by Abbott Laboratories, North Chicago, IL 60064).

Neutralizing antibodies and antibodies mediating antibody-dependent cellular cytotoxicity (ADCC) to HIV represent important responses sought (for effective passive immunotherapy and) in an effective HIV vaccine for active immunization. (Weiss, et al., Nature (1985) 316:69-71; Robert-Guroff, et al., supra, 72; Ho, et al., J. Virol. (1987) 61:2024; Cheng-Mayer, et al., Proc. Natl. Acad. Sci. (U.S.A.) (1988) 85:2815; and Rook, et al., J. Immunol. (1987) 138: 1064; Ljunggren, et al., (1987) 139:2263; Ojo-Amaize, et al., 2458; Blumberg, et al., J. Infect. Dis. (1987) 156:878; Shepp, et al., id. (1988) 157:1260; Tyler, et al., V International Conference on AIDS (1989), Abstract No. T.C.O.33, p. 521).

It is significant, therefore, that Clone 3 Antibody, which binds to the gp41 fusion-associated epitope (GCSGKLIC), can both prevent fusion of virus-infected cells and neutralize infectivity of free virus particles, as demonstrated by biological assays presented in Example 12 and Example 19, respectively.

Alternatively, the monoclonal antibody can be bound to a toxin such as deglycosylated ricin A (dgA) chain to form an immunotoxin (IT). Methods for producing immunotoxins of antibodies are well known. The A chain of ricin may be chemically deglycosylated to prevent any immunotoxin subsequently formed from binding to the parenchymal and nonparenchymal cells of the liver through mannose receptors. To produce the derivatized antibody which can then be coupled to dgA, N-succinimidyl 3-(2-pyridyldithio) propionate dissolved in dimethyl-formamide may be added to a solution of the antibody (5 mg/ml) in 0.1 M sodium phosphate buffer with 0.003 M Na₂ EDTA (disodium ethylenediamine tetracetic acid) (pH 7.5) to a final concentration of 1 mM. After 30 minutes at room temperature, the solution may be desalted on a column of Sephadex G-25. The derivatized immunoglobulin may then be added to the dgA chain solution at a ratio of 1.3 mg of deglycosylated A chain to 1 mg of IgG and maintained for 2 hours at 25°C, followed by overnight at 4°C. The resultant IT-dgA (immunotoxin dgA chain-antibody) may then be purified on Sephacryl ACA-44. (Till, et al., Proc. Natl. Acad. Sci. (U.S.A.) (1989) 86:1987-1991).

Only HIV-1-infected cells express viral proteins on their surface. Consequently, only those cells infected with the virus will express gp41. Since the monoclonal antibody of this invention binds to gp41, the monoclonal antibody will be able to target the toxin to only those cells infected with the virus. Additionally, gp41 is a highly conserved peptide, thereby making the therapy described practicable. This particular monoclonal antibody is also able to inhibit cellular fusion between HIV-1-infected and uninfected cells, thus effecting dual purposes when conjugated to the deglycosylated ricin A chain.

Also included within the scope of the invention are useful binding fragments of the described monoclonal antibody, such as Fab, F(ab')₂, and Fv fragments. The antibody fragments are obtained by conventional techniques. Useful binding fragments may be prepared by peptidase digestion of the antibody using papain or pepsin. Consequently, the antibodies, or fragments thereof, may be injected either with or without a conjugated material for use in treating individuals already infected with AIDS.

### Example 14

### Diagnostic Uses for Anti-gp41

Another example of a technique in which the monoclonal antibody and the biological reactive fragments of the invention may be employed is in immunodiagnostic assays involving antigen-antibody reactions. The assays may be homogeneous or heterogeneous. In a homogeneous assay approach, the specimen may be biological fluid such as serum, urine, and the like or the specimen may be lysed and clarified to remove debris. The antibody, for example, can be used to measure the concentration of its cognate antigen in the serum of patients. The immunological reaction usually involves the specific antibody, a labeled analyte, and the sample of interest. The signal arising from the label is modified, directly or indirectly, upon the binding of the antibody to the labeled analyte. Both immunological reaction and detection of the extent thereof are carried out in a homogeneous solution. Immunochemical labels which may be employed include free radicals, fluorescent dyes, enzymes, bacteriophages, coenzymes, and so forth.

In a heterogenous assay approach, the reagents are usually the specimen, the specific antibody, and means for producing a detectable signal. The specimen is generally placed on a support, such as a plate or slide, and contacted with the antibody in a liquid phase. The support is then separated from the liquid phase and either the support phase or the liquid phase is examined for a detectable signal employing means for producing such signal. The signal is related to the presence of the analyte in the specimen. Means for producing a detectable signal includes the use of radioactive labels, fluorescent compounds, enzymes, and so forth. Exemplary heterogeneous immunoassays are the radioimmunoassay, immunofluorescence methods, enzyme-linked immunoassays, and the like.

For a more detailed discussion of the above immunoassay techniques, see "Enzyme-Immunoassay;" by Edward T. Maggio, CRC Press, Inc., Boca Raton, Florida, 1980. See also, for example, U.S. Patent Nos. 3,690,834; 3,791,932; 3,817,837; 3,850,578; 3,853,987; 3,867,517; 3,901,654; 3,935,074; 3,984,533; 3,966,345; and 4,098,876, which listing is not intended to be exhaustive. Methods for conjugating labels to antibodies and antibody fragments are well known in the art. Such methods may be found in U.S. Patent Nos. 4,220,450; 4,235,869; 3,935,974; and 3,966,345. Another example of a technique in which the monoclonal antibody of the invention may be employed is immunoperoxidase labeling. (Sternberger, Immunocytochemistry (1979) pp. 104-169). Alternatively, the monoclonal antibody may be bound to a radioactive material or to a drug to form a radiopharmaceutical or pharmaceutical, respectively. (Carrasquillo, et al., Cancer Treatment Reports (1984) 68:317-328).

### Example 15 (for information only)

### Therapeutic Uses of Fusion-Associated Epitope of Human Immunodeficiency Virus-1 (HIV-1)

With regard to **active immunization,** technology is now available for synthesis of vaccines derived from short amino acid sequences on the surface of a virus or other pathogen. Such small peptide sequences are capable of representing the immunogenic counterpart in the entire pathogen and of stimulating effective neutralizing antibody production. These synthetic vaccines are potentially "medically ideal" reagents, whose clinical applicability has now been studied for almost a decade. (Lerner, R.A., et al., Hospital Practice (1981) 16:55-62, December 1981, Vol. 16, No. 12, pp. 55-62).

The synthetic vaccine approach has been studied in SIV infection in macaques, on the basis of immunological studies with a set of SIV peptides chosen for hydrophilicity and conservation epitopes analogous to HIV gp120 and gp41. The immunogenic potential of a mixture of four of these epitopes (two from gp120 and two from gp41) was tested in macaques. One of the two SIV transmembrane synthetic peptide sequences, corresponding to the HIV-1 transmembrane gp41 amino acid sequence (amino acids 589-609) that contains the neutralizable fusion-associated epitope (amino acids 602-609) considered in this patent as an antigenic/immunogenic peptide of HIV-1, was one of the four subunit synthetic vaccine components utilized to successfully immunize the macaques against SIV challenge.

Schafferman, A., et al., (Proceedings of the National Academy of Science, (U.S.A), (1991), 88:7126-7130, Protection of Macaques with Simian Immunodeficiency Virus (SIV) Envelope Peptide Vaccine Based on Conserved Human Immunodeficiency Virus Type 1 (HIV-1) Sequences), presents an extension and confirmatory update of previous findings, wherein peptides of SIV (two from gp120 and two from gp41, one of which contains a broader sequence that is homologous with the octapeptide of HIV-1 gp41 ascertained and delineated in the present patent application) induced virus-neutralizing and peptide-specific antibodies. After challenge with virulent virus, controls became virus positive with a progressive decrease in CD4+ cell count, while immunized macaques developed a postchallenge anamnestic response to SIVenv antigens by a partial or total suppression of detectable SIV. The average CD4+ cell count in the blood of immunized macaques remained constant, and virus suppression correlated with prechallenge neutralizing antibody titers. The conserved nature of the HIV and SIV peptides which were utilized in the active vaccination protocol for macaques, and the similar humoral, immunoreactivity in the respective hosts suggests that homologous HIV peptides may be important components of a successful immunization strategy.

### PROCEDURE AND DATA OF EXPERIMENTAL PROTOCOL DEMONSTRATING EFFICACY FOR ACTIVE IMMUNOTHERAPY IN VIVO

Further, to the discussion above with regard to protective active immunization in vivo, primate research protocols have demonstrated that vaccination of chimpanzees confers protection against challenge with human immunodeficiency virus (HIV).

Moreover, an active immunization protocol is known in the art as shown in the teachings in Girard, M., et al., Proc. Natl. Acad. Sci. U.S.A., January 1991, 88:542-546, Immunization of Chimpanzees Confers Protection Against Challenge With Human Immunodeficiency Virus. Specifically, the materials and methods are as follows:

### Materials and Methods:

Immunoqens and Immunization Schedule. The antigenic/ immunogenic neutralizable/fusion-associated peptide (epitope) can be synthesized according to the method of Merrifield, modified by Houghten. Before each immunization, the purified peptide is mixed with the Syntex adjuvant formulation 1 (SAF-1 = 1 mg of threonyl muramyl dipeptide), and 2 ml of the mixture is injected intramuscularly (i.m.).

In order to prepare the peptide cross-linked to keyhole limpet hemocyanin (KLH), an aliquot (19.8 mg) of the peptide is treated first with citraconic acid and then coupled to 19.3 mg of keyhole limpet hemocyanin (KLH) by N-terminal tyrosyl linkage using bisdiazobezidine (pH 9.0). After formulation with SAF-1, immunizations with the peptide-conjugate (300 micrograms per dose) are conducted on weeks 0, 6, 10, 33, 38, 66, and 76 by the i.m. route, followed by a series of four injections of free (unconjugated) peptide (100 micrograms per dose) in SAF-1 on weeks 79, 83, 87, and 102.

The described immunization protocol, using a synthetic peptide identical to the major HIV-1 neutralizing epitope V3, has been utilized in the immunization of chimpanzees, wherein the active immunotherapy conferred protection against virus challenge on week 106 (40 chimpanzee infectious doses equivalent to 100 tissue culture 50% infectious doses) with HIV-1. There was a significant increase in neutralizing antibody titers after the V3 peptide inoculation, as demonstrated by inhibition of syncytia formation in CEM-SS cells.

In summary, antibodies directed against epitopes within the V3 loop, elicited by vaccination of chimpanzees with V3 peptide (**and** Clone 3 Antibody directed against the fusion-associated epitope, produced by a human B cell line established from a patient naturally infected with HIV-1), inhibit the fusion-associated function of gp120 (and gp41, respectively) and therefore both antibodies prevent fusion of the viral envelope to the target cell membrane and both antibodies consequently abrogate virus infectivity.

The primates have remained virus negative by serologic and virologic criteria, including polymerase chain reaction (PCR) analysis (through 9 months of follow-up).

These data demonstrate that immune protection can be elicited against infection of HIV-1 by immunization, therefore providing a basis for development of an HIV-1 vaccine. Girard, M., et al., Proc. Natl. Acad. Sci. U.S.C., January 1991, 88:542-546, Immunization of Chimpanzees Confers Protection Against Challenge with Human Immunodeficiency Virus.

Additionally, with regard to **human** (active) immunization protocols, the following data of Redfield, R., et al., were presented recently at the annual meeting sponsored by the Laboratory of Tumor Cell Biology, National Cancer Institute, Bethesda, Md 20892, on September 8, 1991 in the session Vaccines I (Vaccine Therapy Using rgp160 in Early HIV Infection)¹, wherein active immunization in HIV-1 seropositive patients with an HIV envelope derived viral protein product (rgp160) was successfully achieved utilizing a multiple injection series of 6 injections, as demonstrated by humoral and cellular immunogenicity documented in 92% of the volunteers. Of clinical importance, initial association of vaccine responsiveness and lack of CD4 decline persists through 18 months of follow up.
¹ "A Phase I Evaluation of the Safety and Immunogenicity of Vaccination with Recombinant gp160 in Patients With Early Human Immunodeficiency Virus Infection", New England Journal of Medicine, 324: 1677-84, 1991.

With regard to active immunization protocols described for healthy adult (HIV-1 sero-negative) **human** volunteers, seroresponses were detected in 91% by rgp160 enzyme immunoassay (EIA). The details of the (active) vaccination protocol were presented by Viscidi, R., et al., (AIDS Research and Human Retroviruses (1990), 6:1251-1256, Characterization of Serum Antibody Responses to Recombinant HIV-1 gp160 Vaccine by Enzyme Immunoassay).

Other viral pathogens for which synthetic peptide vaccines have been successfully developed include foot-and-mouth disease of cattle (Bittle, J.L., et al., Nature (1982) 298:30; Vaccine 89, Cold Spring Harbor Laboratory Symposium (September 1989) p. 449) and hepatitis B virus in humans, for which a vaccine consists of a single viral protein, the surface antigen prepared by recombinant DNA technology. (Schild, G.C., et al., Lancet (1990) 335:1081).

The active immunization method, utilizing the HIV-1 fusion-associated epitope (peptide) can parallel that delineated by Shafferman, Girard, Redfield, and Viscidi and standardized for hepatitis B, wherein, for example, 20 micrograms of recombinant peptide is the inoculum per each of three inoculations (inoculum = 1 ml) (IM - deltoid muscle), at time intervals of initial, one, and six months after the first dose (e.g., Engerix-B®, a hepatitis B recombinant peptide vaccine, manufactured by SmithKline Biologicals, Rixensart, Belgium, distributed by Smith Kline & French Laboratories, Division of SmithKline Beckman Corporation, Philadelphia, PA 19101).

Because it is not known whether the predominant route of initial HIV-1 infection in humans is by cell-associated virus or cell-free virus, it is important that any vaccine antibody response based on the gp41 fusion-associated epitope (GCSGKLIC (Seq. No. 1)) be able to block both infectious pathways. Again, it is significant, therefore, that Clone 3 Antibody, elicited by the native antigen identified in fine epitope mapping as in the octapeptide represented by the amino acid sequence GCSGKLIC (Seq. No. 1), can both prevent fusion of virus-infected cells and neutralize infectivity of free virus particles, as demonstrated by biological assays presented in Example 12 and Example 19, respectively.

Therefore, therapeutic measures capable of boosting the (decreasing) neutralizing antibody titer of individuals already infected with the human immunodeficiency virus-1 (HIV-1), eliciting high-titer neutralizing antibodies (i.e., active immunotherapy), or increasing (augmenting) neutralizing antibodies (i.e., passive immunotherapy) in individuals at risk would prove beneficial in preventing new infection or in controlling viral spread in vivo, (Robert-Guroff, et al., AIDS Research and Human Retroviruses (1988) 3:343-350), thereby preventing the disease progression to frank AIDS.

### Example 16

### Prognostic Uses of Fusion-Associated Epitope of Human Immunodeficiency Virus-1 (HIV-1) and Anti-gp41 (Clone 3 Antibody)

The peptide can be used as a prognostic tool to measure the concentration of the protecting antibody in the patient's serum. As mentioned previously, there is a correlation between level of protective antibody and the advancement of the disease. As protective antibody level decreases, the disease-state progresses.

Patient samples such as plasma, cerebral spinal fluid, secretions, or excretions may be collected for testing. The samples could then be tested in an in vitro ELISA for quantitating and detecting antibody against the peptide. Microtiter plates coated with the peptide could be used in the screening. The patient samples could be incubated on the peptide-coated plates in various dilutions for times sufficient to allow binding to occur. An anti-human antibody labeled either radioactively or enzymatically for subsequent detection could then be added to the wells. The simultaneous running of a standard curve with known antibody amounts would enable quantitation of the antibody in the patient samples. Additionally, the octapeptide or a portion thereof, could be utilized to immunoadsorb the HIV-1 seropositive sera to isolate and prepare the respective antibody. Handbook of Experimental Immunochemistry in Four Volumes (Ed. D.M. Weir) 4th edition 1986, Aldon Press, Oxford, England, Vol. 1, Chapter 16, Immunoadsorbent, S. Fuchs and M. Sella, pages 16.1-16.5.

Conversely, the human monoclonal antibody can be utilized to detect, capture, monitor, and quantitate the concentration of the respective antigen/virus in the biological fluids listed above, or in a cell-associated state.

AIDS is caused by the retrovirus Human Immunodeficiency Virus-1 (HIV-1). HIV-1 infection is a chronic disease. The time between infection and the development of clinical AIDS probably averages 8 to 10 years. Laboratory tests for Human Immunodeficiency Virus-1 infection are commonly performed for two reasons.

First, diagnostic tests for the detection of the presence of HIV-1 antibodies can determine whether a person has been infected with the virus. Frequently the virus itself is not detected in patients who are seropositive for HIV-1 antibodies. In early stages of the disease most individuals who have been infected with HIV-1 do not develop clinical symptoms caused by the associated immunosuppression that gradually ensues and therefore most infected individuals are asymptomatic with regard to demonstrating signs of the illness.

Second, prognostic tests should be conducted to estimate the stage and activity of HIV-1 disease. Therefore, prognostic tests are important in identifying patients who might benefit from prophylactic therapy against HIV-1 disease progression.

A number of tests have been used to help estimate the rate of disease progression and the stage of disease. Monitoring these laboratory findings are helpful in patient clinical management. Quantitative tests include CD4+ (T-helper) lymphocytes counts, p24 antigen levels, and beta₂-microglobulin levels.

A decrease in average antibody titers (concentration) has been clinically observed in late stages of infection, particularly with regard to antibodies directed against the HIV-1 envelope epitopes and specifically against the TM gp41 region containing the amino acid sequence against which the herein described human monoclonal antibody (Clone 3 Antibody) is biologically reactive. (Shafferman, et al., AIDS Research and Human Retroviruses (1989) 5:33-39; Chiodi, et al., J. Med. Virol. (1987) 23:1-9; McPhee, el al., FEBS Lett. (1988) 233:393-396).

Specifically and significantly, it has been determined recently that a low IgG antibody titer (concentration) against a gp41 peptide (JB7) represented by amino acids CSGKLICTT (Seq. No. 12) (603 - 611, Wain-Hobson/Gnann numbering system) was found to be associated with a rapid disease progression to frank AIDS. (Broliden, et al., AIDS (1989 September) 3:577-582). The clinical correlation and the significant data are presented below: Sera from children that had progressed to frank AIDS (symptomatic) contained almost no antibody reactivity against the indicated region of gp41, CSGKLICTT (Seq. No. 12). Only one out of seven sera from the children with frank AIDS showed antibody reactivity to CSGKLICTT (Seq. No. 12), compared with 12 out of 15 of the rest of the group infected with HIV-1, yet without symptoms of AIDS (asymptomatic).

This significant difference (P less than 0.025, chi square) regarding the concentration of antibodies against the gp41 peptide JB7, could not be seen in peptides representing other parts of gp41, i.e., JB2 and JB4.

The amino acid sequences (single letter abbreviations) are presented for gp41 peptide JB2 and gp41 peptide JB4, for comparison to the amino acid sequence for gp41 peptide JB7, a nine amino acid peptide which contains 7 of the 8 amino acids of the **fusion-associated epitope.** Additionally, the octapeptide amino acid sequence for the gp41 fusion-associated epitope, which contains 7 of the 9 amino acids of the JB7 peptide, is also provided below:

JB2 ⁵⁷⁸IKQLQARILAVERYLKDQQLLGIWG⁶⁰² (Wain-Hobson/Gnann) (Seq. No. 13)

JB4 599GIWGCSGKLICTTAVPWNAS618 (Seq. No. 14)

JB7 603CSGKLICTT611 (Seq. No. 12)

Fusion-Associated Epitope 602GCSGKLIC609 (Seq. No. 1)

However, anti-JB7 (antibody) response probably has no protective function against infection (i.e., absolute prevention of transmission of HIV-1) since the frequency of anti-JB7 (antibody) reactivity in the uninfected group (without HIV-1) was also low. The data indicated that only four sera from 19 of the uninfected children less than 6 months of age reacted with JB7, i.e., demonstrated the presence of anti-JB7 antibodies.

A similar difference of reactivity would probably be possible for identifying other significant (neutralizable) epitopes contained within gp41, with the corresponding (neutralizing) antibodies.

Fusion-Associated Epitope--The peptide can be synthesized according to the solid-phase method of Merrifield (1963) modified by Houghten (1985). For ELISA quantitation of human antibody directed against the fusion-associated epitope, microwell plates (NUNC Immunoplate 1) are coated at 4°C. overnight (or 3 hours at room temperature) with 1 microgram of peptide/microwell. The wells are washed three times with phosphate buffered saline (PBS) and are then incubated with 200 microliters of PBS containing 1% bovine serum albumin (BSA) per well at room temperature for 3 hours to block nonspecific protein binding sites. The wells are then emptied by aspiration, and washed three times with 300 microliters PBS containing 0.05% by volume polyoxyethylene (20) sorbitan monolaurate (e.g., Tween 20, produced by ICI Specialty Chemicals), then twice with 300 microliters of PBS alone. The wells are emptied, dried and the fusion-associated epitope/peptide coated plates are then stored with desiccant in a sealed bag at 4°C.

The test fluid (e.g., blood serum from a human patient or normal individual) is diluted with PBS containing 20% by volume normal goat serum, 1% by weight BSA and 0.05% by volume Tween 20 at dilutions of 1:50 (or 1:500), volume to volume.

In order to quantitate the human antibody in the test sera directed against the fusion-associated epitope, 100 microliters of the diluted sera are added to each well and allowed to react for 2 hours at 37°C. To construct a standard curve in order to calculate the concentrations in test sera of human antibodies directed against the fusion-associated epitope, a known concentration of affinity-chromatography purified Clone 3 Antibody (IgGl) is serially diluted and concomitantly reacted (100 microliters) in the peptide-coated wells. The wells are then washed five times with 0.05% by volume Tween 20 in PBS in order to remove unbound antibodies. A solution of 100 microliters of horseradish peroxidase conjugated goat anti-human IgG at a dilution of 1:100 is used as a second antibody tracer to bind with the human antibody-antigen complex formed in positive wells. After 2 hours at 37°C, the wells are washed three times with 0.05% by volume Tween in PBS, then incubated with 100 microliters of tetramethylbenzidine chromogen in dimethylsulfoxide (DMSO) with buffered hydrogen peroxide substrate for 30 minutes at room temperature. The reaction is stopped by the addition of 100 microliters of 1N sulfuric acid (H₂SO₄) and the optical density or absorbance of the solution determined at 490 nm, a colorimetric determination in the visible spectrum. Assays are performed in duplicate serum samples from normal individuals or from patients with diseases unrelated to HIV-1 infection used as negative controls. Absorbance readings greater than the cutoff value of A₄₉₀ = 0.12, (about 3 times the mean A₄₉₀ value of normal serum control), are recorded as positive.

A prognostic test kit for quantitation of human antibodies directed against the fusion-associated epitope of HIV-1 can be constructed. The test kit comprises a compartmented enclosure containing multiple 96-well plates coated prior to use with 1 microgram per well of the fusion-associated epitope/peptide. The kit further comprises materials for enzyme detection in separate sealed containers consisting of: (1) normal human serum, as negative control; (2) quantitated affinity-chromatography purified Clone 3 Antibody (human monoclonal anti-fusion-associated epitope antibody directed against HIV-1 gp41), as positive control and for construction of a standard curve; (3) normal goat serum; (4) peroxidase labeled-goat antihuman IgG; (5) color change indicator consisting of tetramethylbenzidine chromogen in dimethylsulfoxide (DMSO) with buffered hydrogen peroxide substrate; and (6) 1N sulfuric acid (H₂SO₄). The procedure described above is to be followed.

### Example 17 (for information only)

### Development and Use of Synthetic Inhibitory Peptide

Specifically, in a number of systems synthetic peptides have been derived directly from the amino acid sequences of complementarity-determining regions (CDRs) of the variable domains of antibodies which bind epitopes (antigens) and demonstrated to have binding properties similar to those of the intact antibody. Therefore, the "complementary" amino acid sequence within the Fab fragment of Clone 3 Antibody CDRs that binds to the delineated fusion-associated epitope (GCSGKLIC (Seq. No. 1)), can be determined and synthesized as detailed in the following listed references: Williams, W.V., Guy, H.R., Rubin, D.H., Robey, F., Myers, J.N., Kieber-Emmons, T., Weiner, D.B., Greene, M.I. (1988) Sequence of the Cell Attachment Site of Reovirus Type 3 and Modeling of its Three-Dimensional Structure, Proceedings of the National Academy of Sciences, 85, 6488; Williams, W.V., Moss, D.A., Kieber-Emmons, T., Cohen, J.A., Myers, J.N., Weiner, D.B., Greene, M.I. (1989) Development of Biologically Active Peptides Based on Antibody Structure, Proceedings of the National Academy of Sciences, 86, 5537-5541; and Williams, W.V., Kleber-Emmons, T., VonFeldt, J., Greene, M.I., Weiner, D.B. (1991) Design of Bioactive Peptides Based on Antibody, Hypervariable Region Structures, Development of Conformationally Constrained Peptides with Enhanced Affinity, J. Biol. Chemistry, 266:5182-5190.

Additionally, since the amino acid sequence of the fusion-associated epitope is known, it is possible to synthetically develop a complementary peptide capable of binding to the epitope and thereby capable of blocking fusion. The development of a "synthetic inhibitory peptide" is facilitated in this instance since the tertiary structure of the epitope has been predicted through computer analysis. (Modrow, et al., J. Virol., (1987) 61:570-578; Navia, et al., V International Conference on AIDS (1989) Abstract No. M.C.O.23, p. 513; Debouck, et al., V International Conference on AIDS (1989), Abstract No. T.C.O.11, p. 517).

The amino acid sequence and tertiary structure of the epitope may be input as data into a computer program with 3-dimensional modeling capabilities. Several models of complementary peptides may then be generated. Peptide sequences consisting of the complementary peptides may then be synthesized and tested for fusion inhibition capacity, such as in a syncytium-forming assay. Those peptides found to inhibit fusion may then be produced on a larger scale for therapeutic purposes. Such peptides may be administered orally, intramuscularly, or intravenously.

Specifically, an example of the complementary synthetic inhibitory peptide on gp120 (amino acids 107-134, Myers) that has the biochemical property to form amphipathic helices with three charged amino acid residue contacts (shown below and underscored) and complementary hydrophobic residues on the gp41 (amino acids 584-611, Wain-Hobson/Gnann) and consequently inhibit the function of the fusion-associated epitope on gp41 (amino acid residues 602-609, Wain-Hobson/Gnann), would be, as deduced from the data presented by McPhee, et al., Cold Spring Harbor Symposium (1988), p. 17, and Modrow, S., id.: 107D-I-I-S-L-W-D-Q-S-L-K-P-C-V-K-L-T-P-L-C-V-S-L-K-C-T-D-L134(Myers) (Seq. No. 15) (single letter abbreviations representing amino acid residues 107-134, Myers).

The predicted three charged amino acid residue contacts (shown below and underscored) and the complementary hydrophobic amino acid residues that form part of the putative contact region between gp120 (amino acids 107-134, Myers) and gp41 (amino acids 584-611, Wain-Hobson/Gnann) and the relation to the fusion-associated epitope (shown below in bold type) on gp41 (amino acids 602-609, Wain-Hobson/Gnann), are represented in the comparison below.

gp120 ¹⁰⁵H-E-D-I-I-S-L-W-D-Q-S-L-K-P-C-V-K-L-T-P-L-C-V-S-L-K-C-T-D-L¹³⁴ (Myers) (Seq. No. 16)

gp41 ⁵⁸²Q-A-R-I-L-A-V-E-R-Y-L-K-D-Q-Q-L-L-G-I-W-G-C-S-G-K-L-I-C-T-T⁶¹¹ ⁽ᴳⁿᵃⁿⁿ⁾ (Seq. No. 17)

Thus, with regard to the synthetic inhibitory peptide (neutralizing agent), the complementary synthetic inhibitory peptide sequence may be determined as presented above.

Additional data indicating that the antiviral **synthetic inhibitory peptides** against HIV-1 can be synthesized to mimic structures involved in the contact region between gp120 and gp41 are presented in McPhee, D.A., Vth International Conference on AIDS, Montreal, Canada, June 1989, Abstract T.C.O. 35, Putative Interaction Site Between HIV gp120 and gp41: Antiviral Action of Synthetic Peptides, p. 521.

Synthetic peptides of 8 to 25 amino acid residues were tested by McPhee (at 50-100 micrograms/ml) for their ability to inhibit HIV replication in vitro. A region in gp120 (amino acids 99-119) was identified that inhibited virus replication as demonstrated by lack of increase in reverse transcriptase (RT) activity and delayed syncytial formation. (McPhee, id.).

Specifically, of the peptides tested and reported by McPhee, gp120 (105-117) had marked antiviral activity (99% inhibition of RT activity after 11 days in culture) (FIGURE 2A). (McPhee, D.A., Vaccines 89, Putative Contact Region Between HIV Envelope Proteins gp120 and gp41: Antiviral Action of Synthetic Peptide Analogs, September 1989, pp. 185-189.)

It should also be noted, however, in the reported preliminary experiment that the 25-residue synthetic peptide gp120 (105-129) had **only 50%** inhibition of RT activity after 11 days in culture. (McPhee, id., p. 185).

This synthetic inhibitory peptide (105-129) tested by McPhee did not span entirely the complementary hydrophobic and counterpart gp41 region that contains the **fusion-associated epitope,** as does the synthetic inhibitory peptide **gp120 (107-134)** described and represented above by the Applicant.

### Example 18

### Development and Use of Anti-Idiotypic Antibodies

The human monoclonal antibody Anti-gp41 may be injected as an immunogen into another species, such as mice, in order to raise anti-idiotypic antibodies to the Anti-gp41. Monoclonal anti-idiotypes could also be developed. Anti-idiotypes could be screened for using available assay techniques effecting a competition between the labeled peptide and the potential anti-idiotypes. Those anti-idiotypes capable of displacing the peptide in assay could then be used in place of the peptide for purposes of immunization or diagnosis.

### Example 19

### Neutralization of HIV-1 (SF2) Infectivity by Human Monoclonal Antibody Directed Against gp41

The free virus neutralization assay is conducted as follows: Aliquots of seronegative sera or affinity column (Protein G) purified human monoclonal anti-gp41 antibody (Clone 3 Antibody) (dilutions 1:10 and 1:100) are incubated with HIV-1 (SF2) for 1 hour at 37°C. The virus and antibody sample is then reacted for 3 hours at 37°C with PHA stimulated (3 days) peripheral blood mononuclear cells (PBMC) obtained from normal donors. The supernatant is removed and replaced by growth media after the 3 hour incubation. On day 8 postinfection, the supernatant media (quadruplicates) are assayed for reverse transcriptase (RT) activity.

A human monoclonal antibody (Clone 3 Antibody) concentration of 100 micrograms/ml produced a 96% inhibition of free virus (SF2) infectivity of human mononuclear cells as demonstrated by lack of increase in reverse transcriptase (RT) activity when cell-free supernatant fluids were analyzed on day 8 postinfection (Table 5).

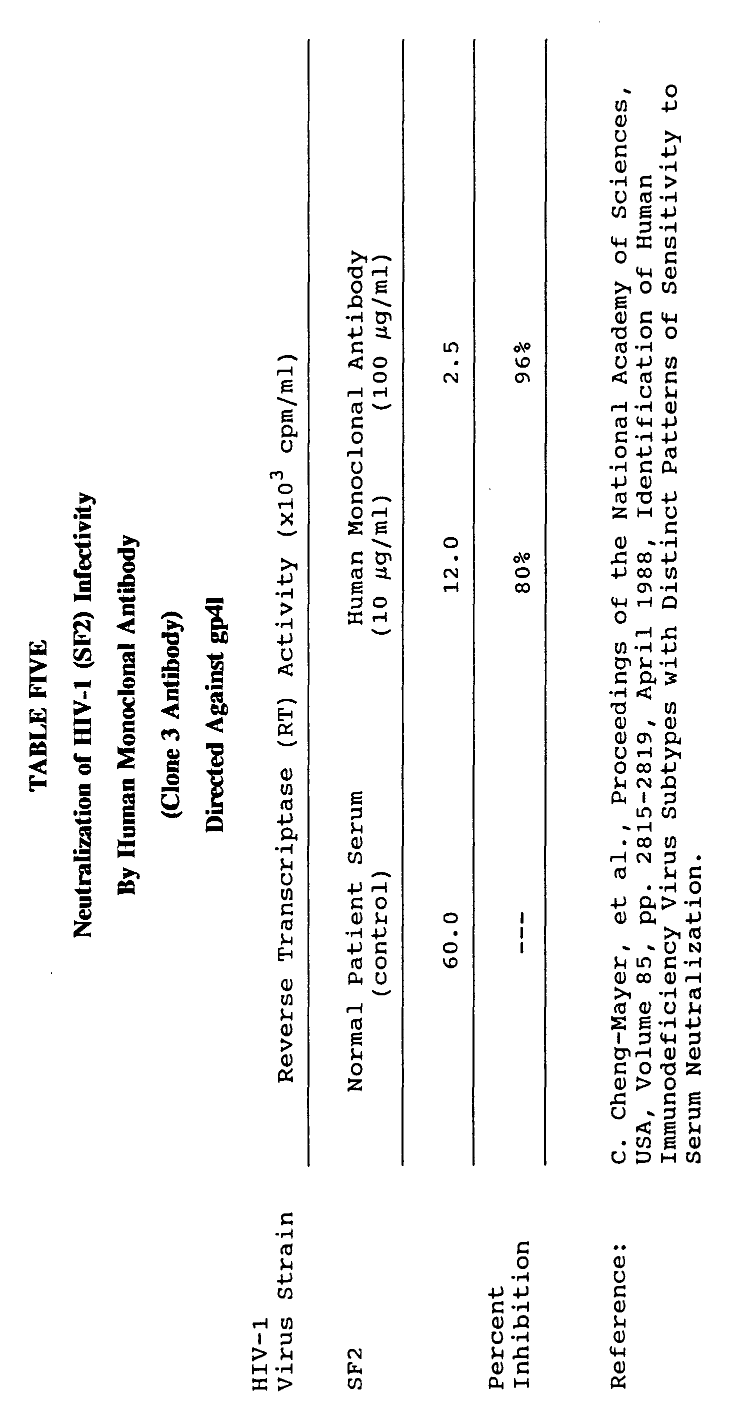

### Example 20

### Immunofluorescence Assay by Flow Cytometry

The indirect immunofluorescence staining procedure was performed as described below.

Aliquots of live Sup-T1 cells (10⁶ in 50 microliters PBS with 0.1% BSA and 0.02% sodium azide), either HTLV-IIIB.9 infected or uninfected, were incubated in parallel procedures with 5 microliters of human monoclonal antibody (Clone 3 Antibody) supernatant for 30 minutes at 4°C. The cells were washed and resuspended in 25 microliters of a 1:40 dilution of affinity-purified fluorescein isothiocyanate (FITC)-conjugated (Fab')₂ goat anti-human .IgG (gamma chain specific) (Tago, Burlingame, CA) for an additional 30 minutes at 4°C. After incubation, the cells were washed repeatedly, fixed in 4% paraformaldehyde, and then ten thousand stained cells were analyzed by flow cytometry using a Becton Dickinson FACS analyzer interfaced to a BD Consort 30 (Becton Dickinson, Mountain View, CA). Cells uninfected by HTLV-IIIB.9 served as a negative control.

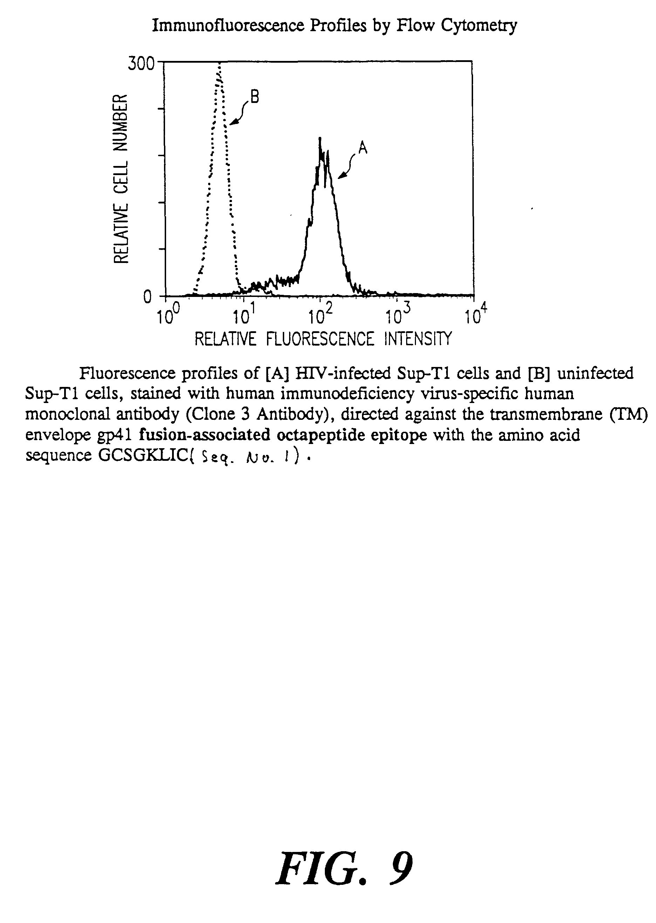

The fluorescence profiles of the binding of human monoclonal antibody (Clone 3 Antibody) to [A] HTLV-IIIB.9 infected Sup-T1 cells (mean relative fluorescence intensity = 127) and [B] uninfected Sup-T1 cells (mean relative fluorescence intensity = 7) as measured by cytofluorographic analysis, are represented in FIGURE 9, wherein relative log green fluorescence intensity (abscissa) is plotted versus relative cell number (ordinate).

The human monoclonal antibody (Clone 3 Antibody) reacted in indirect immunofluorescence assays with a significant proportion of viable HIV-infected cells suggesting that the determinant (epitope) recognized by the antibody is expressed on the surface of HIV-infected cells (and not on uninfected cells) and may be an exposed component of the envelope of HIV.

Therefore, the HIV-specific human monoclonal antibody (Clone 3 Antibody) directed against the transmembrane (TM) envelope gp41 fusion-associated octapeptide epitope with the amino acid sequence GCSGKLIC (Seq. No. 1) (602-609 ^{Wain-Hobson}) demonstrated in cytofluorographic analysis, reactivity to the **native** TM envelope glycoprotein gp41.

These data suggest that the octapeptide with the amino acid sequence GCSGKLIC (Seq. No. 1) (602-609 ^{Wain-Hobson}) represents a native antigenic determinant (epitope) of HIV-1 TM envelope glycoprotein gp41 which is expressed on the surface of HIV-infected cells and also elicits a postinfection immune response with the production of neutralizing antibody, e.g., Clone 3 Antibody.

Although preferred embodiments of the invention have been described in the foregoing Detailed Description, it will be understood that the invention is not limited to the embodiments disclosed, but is capable of numerous rearrangements and modifications without departing from the spirit of the invention. The present invention is therefore intended to encompass such rearrangements and modifications as fall within the scope and spirit of the invention.

## Claims (Claims for the following Contracting State(s): AT, SE, BE, CH, LI, DE, DK, FR, GB, IT, LU, NL)

1. A human monoclonal antibody against an immunodominant peptide of gp41 of HIV-1, which
binds the continuous epitope Gly-Cys-Ser-Gly-Lys-Leu-Ile-Cys of gp41;
neutralizes HIV-1 infectivity through inhibition of syncytium formation and neutralization of free virus
and which is obtainable by a hybridoma cell line having ATCC deposit No. ATCC-CRL-10198.

2. The human monoclonal antibody of claim 1, which requires the amino acid sequence Leu-Ile-Cys as the minimal essential epitope.

3. A Fab, F(ab')₂, or Fv fragment of a monoclonal antibody according to claim 1 or 2.

4. A human hybridoma cell line producing a monoclonal antibody according to claim 1 or 2.

5. A human hybridoma cell line according to claim 4, having the ATCC deposit No. ATCC-CRL-10198.

6. An immunoassay method for the detection and quantitation of HIV infection comprising:
(a) incubating a sample suspected to be infected with HIV with a human monoclonal antibody or a fragment thereof according to any one of claims 1, 2 or 3; and
(b) detecting the binding of the antibody to the sample via immunological methods.

7. An immunoassay method for the detection and quantitation of antibodies directed against gp41 of HIV-1 and prognosis of HIV-1 conditions comprising:
(a) coating a solid support with an effective amount of a peptide composition comprising at least a portion of a peptide selected from the group consisting of:
(I) X-R-Leu-Ile-Cys-R'-Y-Z
where X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or Cys; Z is OH or NH₂; and R is either absent or a sequence of 1-5 amino acids; and R' is either absent or a sequence of 1-2 amino acid; analogues thereof wherein the amino acids in the sequence are substituted as long as the immunoreactivity with antibodies to HIV-1 gp41 derived from the threedimensional conformation of the sequence is substantially preserved; and mixtures and polymers thereof as the antigen, linear as well as cyclic;
(b) adding a test sera, and in parallel testing adding a known and quantitated amount of a human monoclonal antibody or a fragment thereof according to any one of claims 1, 2 or 3 diluted with a buffer wherein the antibodies to the peptide in the test sera and the quantitated human monoclonal antibody standard control form a peptide-antibody complex with the peptide composition;
(c) incubating the mixture at an appropriate temperature and time;
(d) detecting the presence of the peptide-antibody complex and determining the concentration of the specific antibody via the generated standard curve; and
(e) making a good prognosis that said patient is unlikely to develop the symptoms of AIDS based upon a finding of an amount of an antibody binding to the Peptide (I), and a poor prognosis that said patient is likely to develop the symptoms of AIDS based upon a finding of no antibody finding to the Peptide (I).

8. An immunoassay method according to claim 7 wherein the solid support is coated with the peptide in an amount of 0.1-20 micrograms.

9. An immunoassay method according to any one of claims 7 or 8 wherein step (d) comprises introducing a second known antibody labeled with an enzyme and a substrate which reacts with the enzyme to form a colored product.

10. An immunoassay method according to any one of claims 7 or 8 wherein step (d) comprises introducing a second known antibody labeled with a radioactive element.

11. A test kit for the detection and quantitation of antibodies directed against gp 41 of HIV-1 and prognosis of HIV-1 condition comprising:
(a) a solid support;
(b) coated onto the solid support, an immunoabsorbent comprising a peptide composition according to claim 6(a):
(c) a sample of normal serum as negative control;
(d) a sample of quantitated affinity-chromatography purified human monoclonal antibody or a fragment thereof according to any one of claims 1, 2 or 3, as positive control and for construction of a standard curve;
(e) a buffer for diluting the test fluid (e.g. serum samples) the antibody; and
(f) a second antibody tracer to bind with the antibody-antigen complex formed in the immunoassay.

12. A method for neutralizing the retrovirus HIV-1 in vitro, comprising adding to a mixture of HIV-1 virus and uninfected cells, an effective amount of a monoclonal antibody or a fragment thereof according to any one of claims 1, 2 or 3 under conditions effective for allowing said neutralizing agent to neutralize HIV-1.

13. A method for producing a pharmaceutical composition for effecting passive immunization comprising the step of mixing a monoclonal antibody or a fragment thereof according to any one of claims 1, 2 or 3 with a pharmaceutically acceptable carrier.

14. A method for producing a pharmaceutical composition for effecting antiviral therapy through selective cellular toxicity comprising conjugating of a cytotoxic agent to a monoclonal antibody or a fragment thereof according to any one of claims 1, 2 or 3.

15. A method for producing an anti-idiotipic antibody comprising the step of injecting an immunogenic amount of a monoclonal antibody or a fragment thereof according to any one of claims 1 to 3 into an animal.

## Claims (Claims for the following Contracting State(s): ES, GR)

1. A human monoclonal antibody against an immunodominant peptide of gp41 of HIV-1, which
binds the continuous epitope Gly-Cys-Ser-Gly-Lys-Leu-Ile-Cys of gp41;
neutralizes HIV-1 infectivity through inhibition of syncytium formation and neutralization of free virus
and which is obtainable by a hybridoma cell line having ATCC deposit No. ATCC-CRL-10198.

2. The human monoclonal antibody of claim 1, which requires the amino acid sequence Leu-Ile-Cys as the minimal essential epitope.

3. A Fab, F(ab')₂, or Fv fragment of a monoclonal antibody according to claim 1 or 2.

4. A human hybridoma cell line producing a monoclonal antibody according to claim 1 or 2.

5. A human hybridoma cell line according to claim 4, having the ATCC deposit No. ATCC-CRL-10198.

6. An immunoassay method for the detection and quantitation of HIV infection comprising:
(a) incubating a sample suspected to be infected with HIV with a human monoclonal antibody or a fragment thereof according to any one of claims 1, 2 or 3; and
(b) detecting the binding of the antibody to the sample via immunological methods.

7. An immunoassay method for the detection and quantitation of antibodies directed against gp41 of HIV-1 and prognosis of HIV-1 conditions comprising:
(a) coating a solid support with an effective amount of a peptide composition comprising at least a portion of a peptide selected from the group consisting of:
(I) X-R-Leu-Ile-Cys-R'-Y-Z
where X is either a H of the amino terminal NH₂ group of the peptide or an additional amino acid bonded to the amino terminal NH₂ group of the peptide, the additional amino acid being selected to facilitate coupling of the peptide to a carrier protein; Y is absent or Cys; Z is OH or NH₂; and R is either absent or a sequence of 1-5 amino acids; and R' is either absent or a sequence of 1-2 amino acid; analogues thereof wherein the amino acids in the sequence are substituted as long as the immunoreactivity with antibodies to HIV-1 gp41 derived from the threedimensional conformation of the sequence is substantially preserved; and mixtures and polymers thereof as the antigen, linear as well as cyclic;
(b) adding a test sera, and in parallel testing adding a known and quantitated amount of a human monoclonal antibody or a fragment thereof according to any one of claims 1, 2 or 3 diluted with a buffer wherein the antibodies to the peptide in the test sera and the quantitated human monoclonal antibody standard control form a peptide-antibody complex with the peptide composition;
(c) incubating the mixture at an appropriate temperature and time;
(d) detecting the presence of the peptide-antibody complex and determining the concentration of the specific antibody via the generated standard curve; and
(e) making a good prognosis that said patient is unlikely to develop the symptoms of AIDS based upon a finding of an amount of an antibody binding to the Peptide (I), and a poor prognosis that said patient is likely to develop the symptoms of AIDS based upon a finding of no antibody finding to the Peptide (I).

8. An immunoassay method according to claim 7 wherein the solid support is coated with the peptide in an amount of 0.1-20 micrograms.

9. An immunoassay method according to any one of claims 7 or 8 wherein step (d) comprises introducing a second known antibody labeled with an enzyme and a substrate which reacts with the enzyme to form a colored product.

10. An immunoassay method according to any one of claims 7 or 8 wherein step (d) comprises introducing a second known antibody labeled with a radioactive element.

11. A test kit for the detection and quantitation of antibodies directed against gp 41 of HIV-1 and prognosis of HIV-1 condition comprising:
(a) a solid support;
(b) coated onto the solid support, an immunoabsorbent comprising a peptide composition according to claim 6(a):
(c) a sample of normal serum as negative control;
(d) a sample of quantitated affinity-chromatography purified human monoclonal antibody or a fragment thereof according to any one of claims 1, 2 or 3, as positive control and for construction of a standard curve;
(e) a buffer for diluting the test fluid (e.g. serum samples) the antibody; and
(f) a second antibody tracer to bind with the antibody-antigen complex formed in the immunoassay.

12. A method for neutralizing the retrovirus HIV-1 in vitro, comprising adding to a mixture of HIV-1 virus and uninfected cells, an effective amount of a monoclonal antibody or a fragment thereof according to any one of claims 1, 2 or 3 under conditions effective for allowing said neutralizing agent to neutralize HIV-1.

13. A method for producing a pharmaceutical composition for effecting passive immunization comprising the step of mixing a monoclonal antibody or a fragment thereof according to any one of claims 1, 2 or 3 with a pharmaceutically acceptable carrier.

14. A method for producing a pharmaceutical composition for effecting antiviral therapy through selective cellular toxicity comprising conjugating of a cytotoxic agent to a monoclonal antibody or a fragment thereof according to any one of claims 1, 2 or 3.

15. A method for producing an anti-idiotipic antibody comprising the step of injecting an immunogenic amount of a monoclonal antibody or a fragment thereof according to any one of claims 1 to 3 into an animal.

16. A method for producing human monoclonal antibodies against an immunodominant peptide of gp41 of HIV-1, which
bind the continuous epitope Gly-Cys-Ser-Gly-Lys-Leu-Ile-Cys of gp41, and
neutralize HIV-1 infectivity through inhibition of syncytium formation and neutralization of free virus,
comprising cultivating hybridoma cell line having ATCC deposit No. ATCC-CRL-10198 and obtaining said antibodies from the culture supernatant.

17. The method of claim 16, wherein the antibody requires the amino acid sequence Leu-Ile-Cys as the minimal essential epitope.

18. A method for producing Fab, F(ab')₂, or Fv fragments of a monoclonal antibody according to claim 1 or 2, comprising performing the method of claim 16 or 17 and further comprising the step of obtaining said Fab, F(ab')₂, or Fv fragments.

## Patentansprüche (Patentansprüche für folgende(n) Vertragsstaat(en): AT, BE, CH, LI, DE, DK, FR, GB, IT, LU, NL, SE)

1. Humaner monoklonaler Antikörper gegen ein immundominantes Peptid von gp41 von HIV-1, das
an das kontinuierliche Epitop Gly-Cys-Ser-Gly-Lys-Leu-Ile-Cys von gp41 bindet, der
HIV-1-Infektivität durch Inhibierung der Syncytium-Bildung und Neutralisation des freien Virus neutralisiert und der
von einer Hybridomzellinie mit der ATCC-Hinterlegungsnummer ATCC-CRL-10198 erhältlich ist.

2. Humaner monoklonaler Antikörper nach Anspruch 1, der die Aminosäuresequenz Leu-Ile-Cys als das minimale essentielle Epitop erfordert.

3. Ein Fab, F(ab')₂ oder Fv Fragment eines monoklonalen Antikörpers nach Anspruch 1 oder 2.

4. Humane Hybridomzellinie, die einen monoklonalen Antikörper nach Anspruch 1 oder 2 produziert.

5. Humane Hybridomzellinie nach Anspruch 4, die die ATCC-Hinterlegungsnummer ATCC-CRL-10198 besitzt.

6. Immuncassay-Verfahren zum Nachweis und zur Quantifizierung einer HIV-Infektion, das folgende Schritte umfaßt:
(a) Inkubieren einer Probe, von der man annimmt, daß sie mit HIV infiziert ist, mit einem humanen monoklonalen Antikörper oder einem Fragment davon nach irgendeinem der Ansprüche 1, 2 oder 3; und
(b) Nachweisen der Bindung des Antikörpers an die Probe mittels immunologischer Methoden.

7. Ein Imunoassay-Verfahren zum Nachweis und zur Quantifizierung von Antikörpern, die gegen gp41 von HIV-1 gerichtet sind und zur Prognose von HIV-1-Zuständen, das folgende Schritte umfaßt:
(a) Beschichtung eines festen Trägers mit einer effektiven Menge einer Peptid-Zusammensetzung, die wenigstens einen Teil eines Peptids umfaßt, das aus der Gruppe bestehend aus:
(I) X-R-Leu-Ile-Cys-R'-Y-Z
ausgewählt ist, wobei X entweder ein H der aminoterminalen NH₂-Gruppe des Peptids oder eine zusätzliche Aminosäure ist, die an die aminoterminale NH₂-Gruppe des Peptids gebunden ist, wobei die zusätzliche Aminosäure ausgewählt ist, um die Koppelung des Peptids an ein Trägerprotein zu erleichtern; Y ist abwesend oder Cys; Z ist OH oder NH₂; und R ist entweder abwesend oder eine Sequenz von 1-5 Aminosäuren; und R' ist entweder abwesend oder eine Sequenz von 1-2 Aminosäuren; Analoga davon, wobei die Aminosäuren in der Sequenz substituiert sind, soweit die Immunoreaktivität mit Antikörpern gegen HIV-1 gp41, abgeleitet von der dreidimensionalen Konformation der Sequenz, im wesentlichen erhalten bleibt; und Mischungen und Polymere davon als Antigen, linear ebenso wie zyklisch;
(b) Zugeben von Testsera und in parallelen Untersuchungen Zugeben einer bekannten und quantifizierten Menge eines humanen monoklonalen Antikörpers oder eines Fragments davon nach irgendeinem der Ansprüche 1, 2 oder 3, verdünnt mit einem Puffer, wobei die Antikörper gegen das Peptid in den Testseren und dem quantifizierten humanen monoklonalen Antikörperstandardprotokoll einen Peptid-Antikörper-Komplex mit der Peptidzusammensetzung bilden;
(c) Inkubieren der Mischung bei/für geeignete(r) Temperatur und Zeit;
(d) Nachweisen des Vorliegens des Peptid-Antikörper-Komplexes und Bestimmung der Konzentration des spezifischen Antikörpers gegen die erzeugte Standardkurve; und
(e) Anstellen einer guten Prognose, daß der Patient höchstwahrscheinlich keine AIDS-Symptome entwickelt, basierend auf dem Befund, daß eine Menge eines Antikörpers an das Peptid (I) bindet, und Anstellen einer schlechten Prognose, daß der Patient höchstwahrscheinlich die AIDS-Symptome entwickelt, basierend auf einem Befund, daß keine Antikörper zum Peptid (I) finden.

8. Immunoassay-Verfahren nach Anspruch 7, bei dem der feste. Träger mit dem Peptid in einer Menge von 0,1-20 µg beschichtet ist.

9. Immunoassay-Verfahren nach irgendeinem der Ansprüche 7 oder 8, bei dem Stufe (d) das Einführen eines zweiten bekannten Antikörpers, der mit einem Enzym markiert ist, und eines Substrats, das mit dem Enzym unter Bildung eines farbigen Produkts reagiert, umfaßt.

10. Immunoassay-Verfahren nach irgendeinem der Ansprüche 7 oder 8, bei dem Stufe (d) das Einführen eines zweiten bekannten Antikörpers umfaßt, der mit einem radioaktiven Element markiert ist.

11. Testkit zum Nachweis und zur Quantifizierung von Antikörpern, die gegen gp41 von HIV-1 gerichtet sind, und zur Prognose eines HIV-1-Zustands, das folgendes umfaßt:
(a) einen festen Träger;
(b) auf den festen Träger beschichtetes Immunoabsorbens, das eine Peptidzusammensetzung nach Anspruch 6(a) umfaßt;
(c) eine Probe eines Normalserums als Negativkontrolle;
(d) eine Probe eines quantifizierten Affinitätschromatographie-gereinigten humanen monoklonalen Antikörpers oder eines Fragments davon nach irgendeinem der Ansprüche 1, 2 oder 3 als Positivkontrolle und zur Erzeugung einer Standardkurve;
(e) einen Puffer zum Verdünnen der Testflüssigkeit (z.B. Serumproben) des Antikörpers; und
(f) einen zweiten Antikörper-Tracer zum Binden an den im Immunoassay gebildeten Antikörper-Antigen-Komplex.

12. Verfahren zum Neutralisieren des Retrovirus HIV-1 in vitro, das das Zugeben einer Mischung von HIV-1-Virus und uninfizierten Zellen, einer effektiven Menge eines monoklonalen Antikörpers oder eines Fragments davon nach irgendeinem der Ansprüche 1, 2 oder 3 unter Bedingungen umfaßt, die bewirken, daß es dem Neutralisierungsagens ermöglicht ist, HIV-1 zu neutralisieren.

13. Verfahren zur Herstellung einer pharmazeutischen Zusammensetzung zur Erzielung einer passiven Immunisierung, das den Schritt des Vermischens eines monoklonalen Antikörpers oder eines Fragments davon nach irgendeinem der Ansprüche 1, 2 oder 3 mit einem pharmazeutisch annehmbaren Träger umfaßt.

14. Verfahren zur Herstellung einer pharmazeutischen Zusammensetzung zur Erzielung einer antiviralen Therapie durch selektive zelluläre Toxizität, das das Konjugieren eines zytotoxischen Agens an einen monoklonalen Antikörper oder ein Fragment davon nach irgendeinem der Ansprüche 1, 2 oder 3 umfaßt.

15. Verfahren zur Herstellung eines Anti-Idiotypen-Antikörpers, das das Injizieren einer immunogenen Menge eines monoklonalen Antikörpers oder eines Fragments davon nach irgendeinem der Ansprüche 1 bis 3 in ein Tier umfaßt.

## Patentansprüche (Patentansprüche für folgende(n) Vertragsstaat(en): ES, GR)

1. Humaner monoklonaler Antikörper gegen ein immundominantes Peptid von gp41 von HIV-1, das
an das kontinuierliche Epitop Gly-Cys-Ser-Gly-Lys-Leu-Ile-Cys von gp41 bindet, der
HIV-1-Infektivität durch Inhibierung der Syncytium-Bildung und Neutralisation des freien Virus neutralisiert und der
von einer Hybridomzellinie mit der ATCC-Hinterlegungsnummer ATCC-CRL-10198 erhältlich ist.

2. Humaner monoklonaler Antikörper nach Anspruch 1, der die Aminosäuresequenz Leu-Ile-Cys als das minimale essentielle Epitop erfordert.

3. Ein Fab, F(ab')₂ oder Fv Fragment eines monoklonalen Antikörpers nach Anspruch 1 oder 2.

4. Humane Hybridomzellinie, die einen monoklonalen Antikörper nach Anspruch 1 oder 2 produziert.

5. Humane Hybridomzellinie nach Anspruch 4, die die ATCC-Hinterlegungsnummer ATCC-CRL-10198 besitzt.

6. Immunoassay-Verfahren zum Nachweis und zur Quantifizierung einer HIV-Infektion, das folgende Schritte umfaßt:
(a) Inkubieren einer Probe, von der man annimmt, daß sie mit HIV infiziert ist, mit einem humanen monoklonalen Antikörper oder einem Fragment davon nach irgendeinem der Ansprüche 1, 2 oder 3; und
(b) Nachweisen der Bindung des Antikörpers an die Probe mittels immunologischer Methoden.

7. Ein Immunoassay-Verfahren zum Nachweis und zur Quantifizierung von Antikörpern, die gegen gp41 von HIV-1 gerichtet sind und zur Prognose von HIV-1-Zuständen, das folgende Schritte umfaßt:
(a) Beschichtung eines festen Trägers mit einer effektiven Menge einer Peptid-Zusammensetzung, die wenigstens einen Teil eines Peptids umfaßt, das aus der Gruppe bestehend aus:
(I) X-R-Leu-Ile-Cys-R'-Y-Z
ausgewählt ist, wobei X entweder ein H der aminoterminalen NH₂-Gruppe des Peptids oder eine zusätzliche Aminosäure ist, die an die aminoterminale NH₂-Gruppe des Peptids gebunden ist, wobei die zusätzliche Aminosäure ausgewählt ist, um die Koppelung des Peptids an. ein Trägerprotein zu erleichtern; Y ist abwesend oder Cys; Z ist OH oder NH₂; und R ist entweder abwesend oder eine Sequenz von 1-5 Aminosäuren; und R' ist entweder abwesend oder eine Sequenz von 1-2 Aminosäuren; Analoga davon, wobei die Aminosäuren in der Sequenz substituiert sind, soweit die Immunoreaktivität mit Antikörpern gegen HIV-1 gp41, abgeleitet von der dreidimensionalen Konformation der Sequenz, im wesentlichen erhalten bleibt; und Mischungen und Polymere davon als Antigen, linear ebenso wie zyklisch;
(b) Zugeben von Testsera und in parallelen Untersuchungen Zugeben einer bekannten und quantifizierten Menge eines humanen monoklonalen Antikörpers oder eines Fragments davon nach irgendeinem der Ansprüche L, 2 oder 3, verdünnt mit einem Puffer, wobei die Antikörper gegen das Peptid in den Testseren und dem quantifizierten humanen monoklonalen Antikörperstandardprotokoll einen Peptid-Antikörper-Komplex mit der Peptidzusammensetzung bilden;
(c) Inkubieren der Mischung bei/für geeignete(r) Temperatur und Zeit;
(d) Nachweisen des Vorliegens des Peptid-Antikörper-Komplexes und Bestimmung der Konzentration des spezifischen Antikörpers gegen die erzeugte Standardkurve; und
(e) Anstellen einer guten Prognose, daß der Patient höchstwahrscheinlich keine AIDS-Symptome entwickelt, basierend auf dem Befund, daß eine Menge eines Antikörpers an das Peptid (I) bindet, und Anstellen einer schlechten Prognose, daß der Patient höchstwahrscheinlich die AIDS-Symptome entwickelt, basierend auf einem Befund, daß keine Antikörper zum Peptid (I) finden.

8. Immunoassay-Verfahren nach Anspruch 7, bei dem der feste. Träger mit dem Peptid in einer Menge von 0,1-20 µg beschichtet ist.

9. Immunoassay-Verfahren nach irgendeinem der Ansprüche 7 oder 8, bei dem Stufe (d) das Einführen eines zweiten bekannten Antikörpers, der mit einem Enzym markiert ist, und eines Substrats, das mit dem Enzym unter Bildung eines farbigen Produkts reagiert, umfaßt.

10. Immunoassay-Verfahren nach irgendeinem der Ansprüche 7 oder 8, bei dem Stufe (d) das Einführen eines zweiten bekannten Antikörpers umfaßt, der mit einem radioaktiven Element markiert ist.

11. Testkit zum Nachweis und zur Quantifizierung von Antikörpern, die gegen gp41 von HIV-1 gerichtet sind, und zur Prognose eines HIV-1-Zustands, das folgendes umfaßt:
(a) einen festen Träger;
(b) auf den festen Träger beschichtetes Immunoabsorbens, das eine Peptidzusammensetzung nach Anspruch 6(a) umfaßt;
(c) eine Probe eines Normalserums als Negativkontrolle;
(d) eine Probe eines quantifizierten Affinitätschromatographie-gereinigten humanen monoklonalen Antikörpers oder eines Fragments davon nach irgendeinem der Ansprüche 1, 2 oder 3 als Positivkontrolle und zur Erzeugung einer Standardkurve;
(e) einen Puffer zum Verdünnen der Testflüssigkeit (z.B. Serumproben) des Antikörpers; und
(f) einen zweiten Antikörper-Tracer zum Binden an den im Immunoassay gebildeten Antikörper-Antigen-Komplex.

12. Verfahren zum Neutralisieren des Retrovirus HIV-1 in vitro, das das Zugeben einer Mischung von HIV-1-Virus und uninfizierten Zellen, einer effektiven Menge eines monoklonalen Antikörpers oder eines Fragments davon nach irgendeinem der Ansprüche 1, 2 oder 3 unter Bedingungen umfaßt, die bewirken, daß es dem Neutralisierungsagens ermöglicht ist, HIV-1 zu neutralisieren.

13. Verfahren zur Herstellung einer pharmazeutischen Zusammensetzung zur Erzielung einer passiven Immunisierung, das den Schritt des Vermischens eines monoklonalen Antikörpers oder eines Fragments davon nach irgendeinem der Ansprüche 1, 2 oder 3 mit einem pharmazeutisch annehmbaren Träger umfaßt.

14. Verfahren zur Herstellung einer pharmazeutischen Zusammensetzung zur Erzielung einer antiviralen Therapie durch selektive zelluläre Toxizität, das das Konjugieren eines zytotoxischen Agens an einen monoklonalen Antikörper oder ein Fragment davon nach irgendeinem der Ansprüche 1, 2 oder 3 umfaßt.

15. Verfahren zur Herstellung eines Anti-Idiotypen-Antikörpers, das das Injizieren einer immunogenen Menge eines monoklonalen Antikörpers oder eines Fragments davon nach irgendeinem der Ansprüche 1 bis 3 in ein Tier umfaßt.

16. Verfahren zur Herstellung humaner monoklonaler Antikörper gegen ein immundominantes Peptid von gp41 von HIV-1, die
an das kontinuierliche Epitop Gly-Cys-Ser-Gly-Lys-Leu-Ile-Cys von gp41 binden, und
HIV-1-Infektivität durch Inhibierung der Syncytium-Bildung und Neutralisation von freiem Virus neutralisieren,
das das Kultivieren der Hybridomzellinie mit der ATCC-Hinterlegungsnummer ATCC-CRL-10198 und das Erhalten der Anti-, körper aus dem Kulturüberstand umfaßt.

17. Verfahren nach Anspruch 16, bei dem der Antikörper die Aminosäuresequenz Leu-Ile-Cys als das minimale essentielle Epitop erfordert.

18. Verfahren zur Herstellung von Fab, F(ab')₂ oder Fv Fragmenten eines monoklonalen Antikörpers nach Anspruch 1 oder 2, das die Durchführung des Verfahrens nach Anspruch 16 oder 17 umfaßt und ferner den Schritt des Erhaltens der Fab, F(ab')₂ oder Fv Fragmente umfaßt.

## Revendications (Revendications pour l'(les) Etat(s) contractant(s) suivant(s): AT, BE, CH, LI, DE, DK, FR, GB, IT, LU, NL, SE)

1. Anticorps monoclonal humain dirigé contre un peptide immunodominant de la glycoprotéine gp41 du VIH-1 qui:
se lie à l'épitope ininterrompu Gly-Cys-Ser-Gly-Lys-Leu-Ile-Cys de gp41 ;
neutralise l'infectiosité du VIH-1 par inhibition de la formation d'un syncytium et neutralisation du virus libre ; et qui
peut être obtenu au moyen d'une lignée cellulaire d'hybridomes déposée auprès de l'ATCC sous le numéro ATCC-CRL-10198.

2. Anticorps monoclonal humain de la revendication 1, qui nécessite la séquence d'acides aminés Leu-Ile-Cys en tant qu'épitope essentiel minimal.

3. Fragment Fab, F(ab')₂ ou Fv d'un anticorps monoclonal selon la revendication 1 ou 2.

4. Lignée cellulaire d'hybridomes humains produisant un anticorps monoclonal selon la revendication 1 ou 2.

5. Lignée cellulaire d'hybridomes humains selon la revendication 4, déposée auprès de l'ATCC sous le numéro ATCC-CRL-10198.

6. Procédé de test immunologique destiné à la détection et au dosage d'une infection par le VIH comprenant :
(a) l'incubation d'un échantillon suspecté d'être infecté par le VIH avec un anticorps monoclonal humain ou un fragment de ce dernier selon l'une quelconque des revendications 1, 2 ou 3 ; et
(b) la détection de la liaison de l'anticorps à l'échantillon au moyen de méthodes immunologiques.

7. Procédé de test immunologique destiné à la détection et au dosage d'anticorps dirigés contre la glycoprotéine gp41 du VIH-1 et pronostic de l'injection par le VIH-1 comprenant les étapes suivantes consistant à :
(a) recouvrir un support solide avec une quantité efficace d'une composition peptidique comprenant au moins une partie d'un peptide choisi au sein du groupe constitué par
(I) X-R-Leu-Ile-Cys-R'-Y-Z
où X est soit un atome d'hydrogène du groupe NH₂ de l'extrémité amino du peptide soit un acide aminé supplémentaire lié au groupe NH₂ de l'extrémité amino du peptide, l'acide aminé supplémentaire étant sélectionné afin de faciliter le couplage du peptide à une protéine de support; Y est absent ou est un résidu Cys ; Z représente un groupe OH ou NH₂ ; et R est soit absent soit une séquence de 1 à 5 acides aminés ; et R' est soit absent soit une séquence de 1 à 2 acides aminés ; des analogues de cette séquence dans lesquels les acides aminés de la séquence sont substitués dans la mesure où l'immunoréactivité avec des anticorps dirigés contre la glycoprotéine gp41 du VIH-1 dérivés de la conformation tridimensionnelle de la séquence est substantiellement conservée ; et des mélanges et polymères de celle-ci en tant qu'antigène, linéaire aussi bien que cyclique ;
b) l'addition de sérums d'essai, et, dans un test parallèle, l'addition d'une quantité connue et dosée d'un anticorps monoclonal humain ou d'un fragment de celui-ci selon l'une quelconque des revendications 1, 2 ou 3 dilué avec un tampon dans lequel les anticorps dirigés contre le peptide dans les sérums d'essai et l'anticorps monoclonal humain dosé témoin de référence forment un complexe peptide-anticorps avec la composition peptidique ;
c) l'incubation du mélange à une température et pendant une durée appropriées ;
d) la détection de la présence du complexe peptide-anticorps et la détermination de la concentration de l'anticorps spécifique au moyen de la courbe étalon établie ;
e) l'établissement d'un bon pronostic selon lequel il est peu probable que ledit patient développe les symptômes du SIDA basé sur l'observation d'une liaison de l'anticorps au peptide (I), et d'un mauvais pronostic selon lequel il est probable que ledit patient développe les symptômes du SIDA basé sur l'observation d'une absence de liaison de l'anticorps au peptide (I).

8. Procédé de test immunologique selon la revendication 7 dans lequel le support solide est recouvert avec le peptide en une quantité de 0,1-20 microgrammes.

9. Procédé de test immunologique selon l'une quelconque des revendications 7 ou 8 dans lequel l'étape (d) comprend l'introduction d'un second anticorps connu marqué avec une enzyme et un substrat qui réagit avec l'enzyme pour former un produit coloré.

10. Procédé de test immunologique selon l'une quelconque des revendications 7 ou 8 dans lequel l'étape (d) comprend l'introduction d'un second anticorps connu marqué avec un élément radioactif.

11. Kit de test de détection et de dosage d'un anticorps dirigé contre la glycoprotéine gp41 du VIH-1 et de pronostic de l'infection par le VIH-1 comprenant
(a) un support solide ;
(b) un immunoadsorbant comprenant une composition peptidique selon la revendication 6(a) recouvrant un support solide ;
(c) un échantillon de sérum normal en tant que témoin négatif ;
(d) un échantillon dosé d'un anticorps monoclonal humain purifié par chromatographie d'affinité ou d'un fragment de celui-ci selon l'une quelconque des revendications 1, 2 ou 3 en tant que témoin positif pour l'établissement d'une courbe étalon ;
(e) un tampon destiné à diluer le fluide d'essai (par exemple les échantillons de sérum) et l'anticorps ; et
(f) un second anticorps traceur qui se lie au complexe anticorps-antigène formé dans le test immunologique.

12. Procédé de neutralisation du rétrovirus VIH-1 *in vitro* comprenant l'addition à un mélange de virus VIH-1 et de cellules non infectées, d'une quantité efficace d'un anticorps monoclonal ou d'un fragment de celui-ci selon l'une quelconque des revendications 1, 2 ou 3 dans des conditions efficaces pour permettre audit agent neutralisant de neutraliser le VIH-1

13. Procédé de préparation d'une composition pharmaceutique destinée à effectuer une immunisation passive comprenant l'étape consistant à mélanger un anticorps monoclonal ou un fragment de celui-ci selon l'une quelconque des revendications 1, 2 ou 3 avec un support pharmaceutiquement acceptable.

14. Procédé de préparation d'une composition pharmaceutique destinée à effectuer un traitement antiviral par toxicité cellulaire sélective comprenant la conjugaison d'un agent cytotoxique avec un anticorps monoclonal ou un fragment de celui-ci selon l'une quelconque des revendications 1, 2 ou 3.

15. Procédé de préparation d'un anticorps anti-idiotypique comprenant l'étape consistant à injecter une quantité immunogène d'un anticorps monoclonal ou d'un fragment de celui ci selon l'une quelconque des revendications 1, 2 ou 3 à un animal.

## Revendications (Revendications pour l'(les) Etat(s) contractant(s) suivant(s): ES, GR)

1. Anticorps monoclonal humain dirigé contre un peptide immunodominant de la glycoprotéine gp41 du VIH-1 qui:
se lie à l'épitope ininterrompu Gly-Cys-Ser-Gly-Lys-Leu-Ile-Cys de gp41 ;
neutralise l'infectiosité du VIH-1 par inhibition de la formation d'un syncytium et neutralisation du virus libre ; et qui
peut être obtenu au moyen d'une lignée cellulaire d'hybridomes déposée auprès de l'ATCC sous le numéro ATCC-CRL-10198.

2. Anticorps monoclonal humain de la revendication 1, qui nécessite la séquence d'acides aminés Leu-Ile-Cys en tant qu'épitope essentiel minimal.

3. Fragment Fab, F(ab')₂ ou Fv d'un anticorps monoclonal selon la revendication 1 ou 2.

4. Lignée cellulaire d'hybridomes humains produisant un anticorps monoclonal selon la revendication 1 ou 2.

5. Lignée cellulaire d'hybridomes humains selon la revendication 4, déposée auprès de l'ATCC sous le numéro ATCC-CRL-10198

6. Procédé de test immunologique destiné à la détection et au dosage d'une infection par le VIH comprenant :
(a) l'incubation d'un échantillon suspecté d'être infecté par le VIH avec un anticorps monoclonal humain ou un fragment de ce dernier selon l'une quelconque des revendications 1, 2 ou 3 ; et
(b) la détection de la liaison de l'anticorps à l'échantillon au moyen de méthodes immunologiques.

7. Procédé de test immunologique destiné à la détection et au dosage d'anticorps dirigés contre la glycoprotéine gp41 du VIH-1 et pronostic de l'injection par le VIH-1 comprenant les étapes suivantes consistant à :
(a) recouvrir un support solide avec une quantité efficace d'une composition peptidique comprenant au moins une partie d'un peptide choisi au sein du groupe constitué par :
(I) X-R-Leu-Ile-Cys-R'-Y-Z
où X est soit un atome d'hydrogène du groupe NH₂ de l'extrémité amino du peptide soit un acide aminé supplémentaire lié au groupe NH₂ de l'extrémité amino du peptide, l'acide aminé supplémentaire étant sélectionné afin de faciliter le couplage du peptide à une protéine de support ; Y est absent ou est un résidu Cys ; Z représente un groupe OH ou NH₂ ; et R est soit absent soit une séquence de 1 à 5 acides aminés ; et R' est soit absent soit une séquence de 1 à 2 acides aminés ; des analogues de cette séquence dans lesquels les acides aminés de la séquence sont substitués dans la mesure où l'immunoréactivité avec des anticorps dirigés contre la glycoprotéine gp41 du VIH-1 dérivés de la conformation tridimensionnelle de la séquence est substantiellement conservée ; et des mélanges et polymères de celle-ci en tant qu'antigène, linéaire aussi bien que cyclique ;
b) l'addition de sérums d'essai, et, dans un test parallèle, l'addition d'une quantité connue et dosée d'un anticorps monoclonal humain ou d'un fragment de celui-ci selon l'une quelconque des revendications 1, 2 ou 3 dilué avec un tampon dans lequel les anticorps dirigés contre le peptide dans les sérums d'essai et l'anticorps monoclonal humain dosé témoin de référence forment un complexe peptide-anticorps avec la composition peptidique ;
c) l'incubation du mélange à une température et pendant une durée appropriées ;
d) la détection de la présence du complexe peptide-anticorps et la détermination de la concentration de l'anticorps spécifique au moyen de la courbe étalon établie ;
e) l'établissement d'un bon pronostic selon lequel il est peu probable que ledit patient développe les symptômes du SIDA basé sur l'observation d'une liaison de l'anticorps au peptide (I), et d'un mauvais pronostic selon lequel il est probable que ledit patient développe les symptômes du SIDA basé sur l'observation d'une absence de liaison de l'anticorps au peptide (I).

8. Procédé de test immunologique selon la revendication 7 dans lequel le support solide est recouvert avec le peptide en une quantité de 0,1-20 microgrammes.

9. Procédé de test immunologique selon l'une quelconque des revendications 7 ou 8 dans lequel l'étape (d) comprend l'introduction d'un second anticorps connu marqué avec une enzyme et un substrat qui réagit avec l'enzyme pour former un produit coloré.

10. Procédé de test immunologique selon l'une quelconque des revendications 7 ou 8 dans lequel l'étape (d) comprend l'introduction d'un second anticorps connu marqué avec un élément radioactif.

11. Kit de test de détection et de dosage d'un anticorps dirigé contre la glycoprotéine gp41 du VIH-1 et de pronostic de l'infection par le VIH-1 comprenant
(a) un support solide ;
(b) un immunoadsorbant comprenant une composition peptidique selon la revendication 6(a) recouvrant un support solide ;
(c) un échantillon de sérum normal en tant que témoin négatif ;
(d) un échantillon dosé d'un anticorps monoclonal humain purifié par chromatographie d'affinité ou d'un fragment de celui-ci selon l'une quelconque des revendications 1, 2 ou 3 en tant que témoin positif pour l'établissement d'une courbe étalon ;
(e) un tampon destiné à diluer le fluide d'essai (par exemple les échantillons de sérum) et l'anticorps ; et
(f) un second anticorps traceur qui se lie au complexe anticorps-antigène formé dans le test immunologique.

12. Procédé de neutralisation du rétrovirus VIH-1 *in vitro* comprenant l'addition à un mélange de virus VIH-1 et de cellules non infectées, d'une quantité efficace d'un anticorps monoclonal ou d'un fragment de celui-ci selon l'une quelconque des revendications 1, 2 ou 3 dans des conditions efficaces pour permettre audit agent neutralisant de neutraliser le VIH-1.

13. Procédé de préparation d'une composition pharmaceutique destinée à effectuer une immunisation passive comprenant l'étape consistant à mélanger un anticorps monoclonal ou un fragment de celui-ci selon l'une quelconque des revendications 1, 2 ou 3 avec un support pharmaceutiquement acceptable.

14. Procédé de préparation d'une composition pharmaceutique destinée à effectuer un traitement antiviral par toxicité cellulaire sélective comprenant la conjugaison d'un agent cytotoxique avec un anticorps monoclonal ou un fragment de celui-ci selon l'une quelconque des revendications 1, 2 ou 3.

15. Procédé de préparation d'un anticorps anti-idiotypique comprenant l'étape consistant à injecter une quantité immunogène d'un anticorps monoclonal ou d'un fragment de celui ci selon l'une quelconque des revendications 1, 2 ou 3 à un animal.

16. Procédé de préparation des anticorps monoclonaux humains dirigé contre un peptide immunodominant de la glycoprotéine gp41 du VIH-1, qui
lient à l'épitope ininterrompu Gly-Cys-Ser-Gly-Lys-Leu-Ile-Cys de gp41,
neutralisent l'infectiosité du VLH-1 par inhibition de la formation d'un syncytium et neutralisation du virus libre,
comprenant l'étape consistant à cultiver du lignée cellulaire d'hybridomes déposée auprès de l'ATCC sous le numéro ATCC-CRL-10189 et l'étape consistant à recueillir l'anticorps à partir du surnageant.

17. Procédé de la revendication 16 dans lequel l'anticorps nécessite la séquence d'acides aminés Leu-Ile-Cys en tant qu'épitope essentiel minimal.

18. Procédé de préparation des fragments Fab, F(ab')₂ ou Fv d'un anticorps monoclonal humain selon la revendication 1 ou 2, comprenant l'étape consistant à l'exécution du procédé selon revendication 16 ou 17 et comprenant l'étape consistant à l'obtenir des fragments Fab, F(ab')₂ ou Fv.
